# EUROPEAN PATENT APPLICATION

(11) **EP 4 059 937 A1**
(43) Date of publication of application: **21.09.2022**
(21) Application number: 22163076.7
(22) Date of filing: 18.03.2022
(51) Int. Cl.: C07F 7/02, C07F 9/6561, C07D 229/02, C09J 11/06, C09J 185/02

(54) **SYNTHESIS AND USE OF MULTI-FUNCTIONAL DIAZIRINE ADHESIVES FOR ELASTOMER BONDING**

(30) Priority: 19.03.2021 US 202163163309 P
(71) Applicant: Facebook Technologies, LLC, Menlo Park, CA 94025 (US)
(72) Inventor: WALLIN, Thomas John Farrell, Menlo Park, 94025 (US); PAN, Wenyang, Menlo Park, 94025 (US); PURVIS, Lafe, Menlo Park, 94025 (US)
(74) Representative: Murgitroyd & Company

(57) **Abstract**

The present application relates to synthesis and use of multi-functional diazirine adhesives for elastomer bonding. The disclosure provides multi-functional diazirine adhesives and methods of synthesis and use thereof, for example methods of use for polymer and/or elastomer bonding.

## Description

### CROSS REFERENCE TO RELATED APPLICATIONS

This application claims the benefit of, and priority to, U.S. Provisional Patent Application No. 63/163,309, entitled "SYNTHESIS AND USE OF MULTI-FUNCTIONAL DIAZIRINE ADHESIVES FOR ELASTOMER BONDING," filed March 19, 2021, which is incorporated by reference herein in its entirety.

### TECHNICAL FIELD

Described herein are multi-functional diazirine adhesives for elastomer bonding.

### BACKGROUND

Robust, cohesive bonding of chemically dissimilar elastomeric objects remains a challenge to assembling soft robotic and soft wearable devices. The lack of accessible functional groups for crosslinking reactions among conventional soft elastomeric materials prevents suitable covalent grafting between layers. While specific chemistries have been developed for dedicated substrates, these solutions do not apply universally to materials with different chemistries (i.e., polyurethane and polydimethylsiloxane). Recently, bisdiazirines have been identified as suitable for crosslinking (in response to UV light or heat) aliphatic (C-H) groups that are inert to most common adhesives but abundant in many conventional polymer systems. However, this class of bisdiazirines has not been proven to work for heterogeneous bonding (two different substrates) and the difunctional architecture limits utility. For example, there is a threshold concentration above which the bisdiazirine adhesive loses efficacy, likely due to the inability for self-reaction among the adhesive and the inability for a suitable number of molecules to react both diazirine groups to opposing sides of the interface.

Cohesive bonding of soft polymeric (e.g., silicone, polyurethane, etc.) devices to each other, specifically, bonding polymeric actuators and sensors to each other as well as to textiles and wearables remains a challenge. While bisdiazirine molecules are known, tailoring the molecule to desired parameters (number and nature of crosslinking groups, boiling point, melting point, vapor pressure, upper explosive limit, lower explosive limit, solvent compatibility, etc.) can improve adhesive performance. The known bisdiazirine molecules also cannot be commercially sourced and require a complicated synthesis with low yield.

Plasma treatment to induce bondable -OH groups, often stabilized with silane primers, then assembly and heat to drive reaction is known, however this process is not easily implemented on 3D objects, has a strict time window as induced OH groups are temporary, and often yields a weaker bond.

An alternative is to use a specifically designed adhesive for the substrate. This glue usually fails adhesively under tensile load, particularly when combining disparate soft materials.

There remains a need in the art for methods for crosslinking polymeric materials, in particular crosslinking elastomeric polymers.

### SUMMARY OF THE INVENTION

The disclosure provides a compound of Formula I or Formula II: 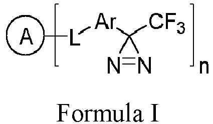 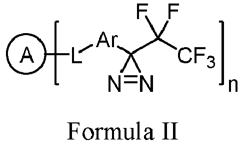 wherein in Formula I and Formula II: A is a core moiety comprising one or more groups selected from optionally substituted alkyl, optionally substituted heteroalkyl, optionally substituted alkenyl, optionally substituted alkynyl, optionally substituted cycloalkyl, optionally substituted heterocycloalkyl, optionally substituted aryl, optionally substituted arylalkyl, optionally substituted heteroaryl, optionally substituted heteroarylalkyl, hydroxy, halo, cyano, trifluoromethyl, trifluoromethoxy, nitro, trimethylsilanyl, optionally substituted epoxide, optionally substituted glycidyl, optionally substituted acrylate, optionally substituted methacrylate, -OR^{a}, -SR^{a}, -OC(O)-R^{a}, -N(R^{a})₂, - C(O)R^{a}, -C(O)OR^{a}, -C(O)SR^{a}, -SC(O)R^{a}, -OC(O)OR^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})_{2,} -N(R^{a})C(NR^{a})N(R^{a})₂, -N(R^{a})S(O)ₜR^{a}, - S(O)ₜR^{a}, -S(O)ₜOR^{a}, -S(O)ₜN(R^{a})₂, -S(O)ₜN(R^{a})C(O)R^{a}, -O(O)P(OR^{a})₂, -O(S)P(OR^{a})₂, P(R^{a}-)₃, and Si, wherein R^{a} is independently selected at each occurrence from hydrogen, optionally substituted alkyl, optionally substituted heteroalkyl, optionally substituted alkenyl, optionally substituted alkynyl, optionally substituted cycloalkyl, optionally substituted heterocycloalkyl, optionally substituted aryl, optionally substituted arylalkyl, optionally substituted heteroaryl, and optionally substituted heteroarylalkyl; L is at each independent occurrence a linker comprising one or more of -Ci-io alkyl-, -O-C₁-₁₀ alkyl-, -Ci-io alkenyl-, -O-C₁-₁₀ alkenyl-, - C₁-₁₀ cycloalkenyl-, -O-C₁-₁₀ cycloalkenyl-, -C₁-₁₀ alkynyl-, -O-C₁-₁₀ alkynyl-, -Ci-io aryl-, -O-C₁-₁₀-, -aryl-, -O-, -S-, -S(O)_{w}-, -C(O)-, -C(O)O-, -OC(O)-, -C(O)S-, -SC(O)-, -OC(O)O-, - N(R^{b})-, -N(R^{b})-C₁-₁₀ alkyl-, -C(O)N(R^{b})-, -N(R^{b})C(O)-, -OC(O)N(R^{b})-, -N(R^{b})C(O)O-, - SC(O)N(R^{b})-, -N(R^{b})C(O)S-, -N(R^{b})C(O)N(R^{b})-, -N(R^{b})C(NR^{b})N(R^{b})-, -N(R^{b})S(O)_{w}-, -S(O)_{w}N(R^{b})-, -S(O)_{w}O-, -OS(O)_{w}-, -OS(O)_{w}O-, -O(O)P(OR^{b})O-, (O)P(O-)₃, - O(S)P(OR^{b})O-, and (S)P(O-)₃, wherein w is 1 or 2, and R^{b} is independently hydrogen, optionally substituted alkyl, or optionally substituted aryl; Ar is at each independent occurrence an optionally substituted aryl substituent, an optionally substituted arylalkyl, an optionally substituted heteroaryl, or an optionally substituted heteroarylalkyl; and n is independently at each occurrence an integer from 0 to 7. In some embodiments, A comprises one or more phenyl groups. In some embodiments, A comprises one or more bi-phenyl groups. In some embodiments, A comprises a group selected from: 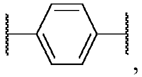 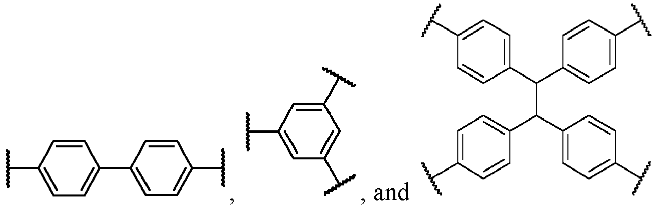 . In some embodiments, A comprises one or more C₁, C₂, C₃, or C₄ alkyl groups. In some embodiments, A comprises one or more Si. In some embodiments, A comprises one or more P. In some embodiments, A comprises one or more groups selected from 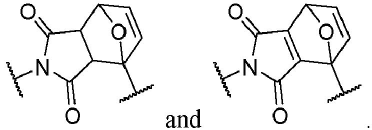 In some embodiments, L comprises one or more groups selected from 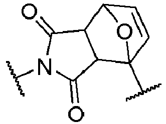 and 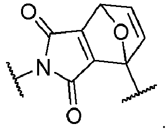 In some embodiments, L comprises one or more C₁, C₂, C₃, or C₄ alkyl groups. In some embodiments, L comprises one or more groups selected from -CH₂-, -CH₂-CH₂-, and -CH₂-CH(CH₃)-. In some embodiments, L comprises one or more groups selected from -O-, -S-, -NH-, -C(O)NH-, -NHC(O)-, -NHC(O)NH-, and -S(O)₂NH-. In some embodiments, Ar comprises any aryl group described herein. In some embodiments, Ar comprises any heteroaryl group described herein. In some embodiments, Ar is selected from 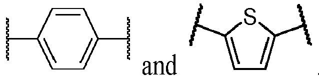 . In some embodiments, the compound has formula 101, 102, 103, or 104: 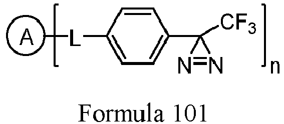 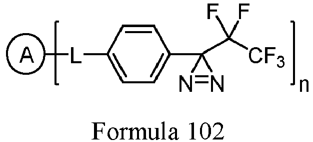 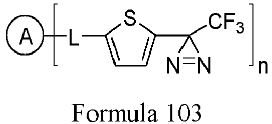 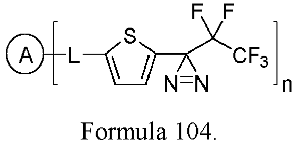 In some embodiments, the compound has Formula 105, 106, 107, 108, 109, 110, 111, 112, 113, 114, 115, or 116: 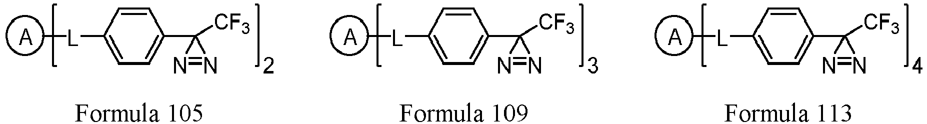 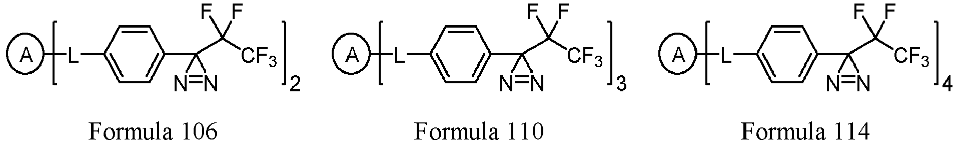 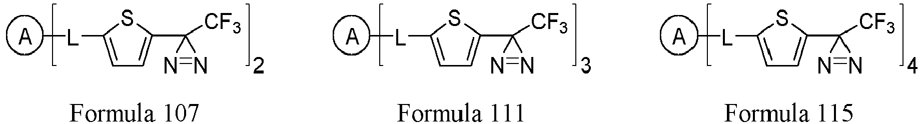 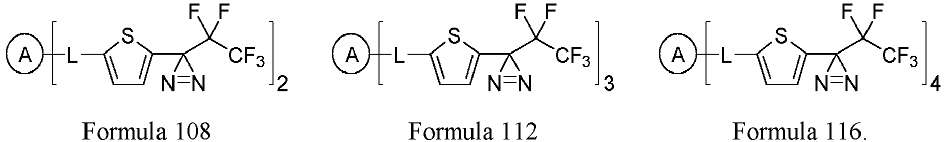 In some embodiments, the compound comprises at least one group selected from CH, CH₂, and CH₃. In some embodiments, the compound comprises at least one CH group. In some embodiments, the compound has any one of Formulas 1001 to 1014:

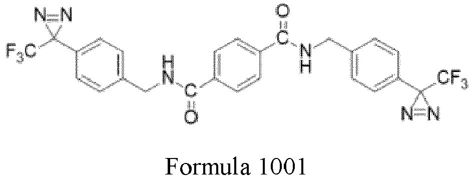

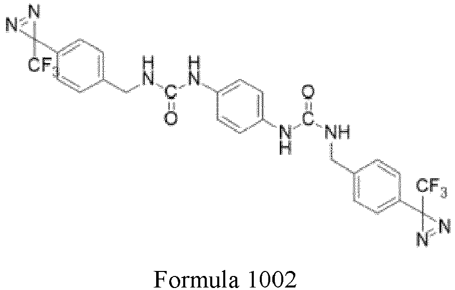

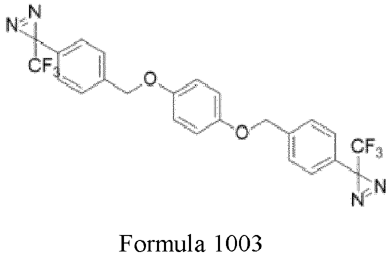

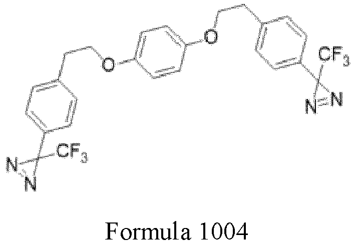

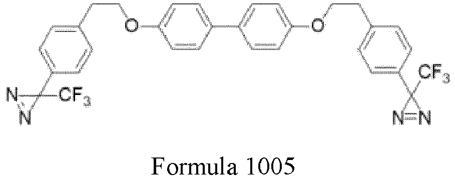

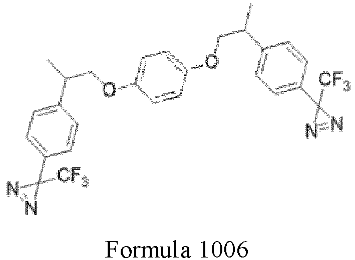

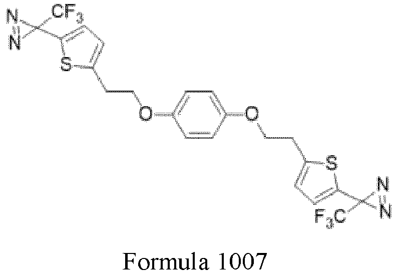

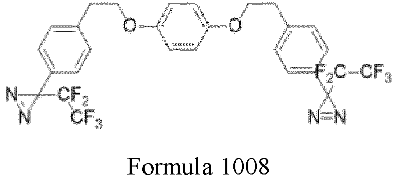

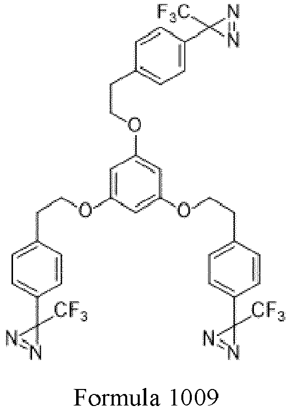

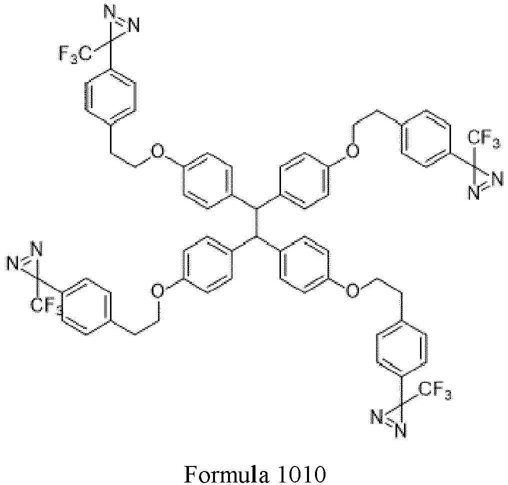

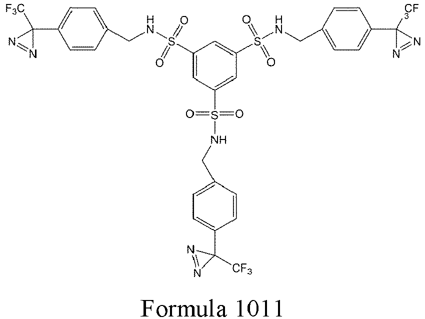

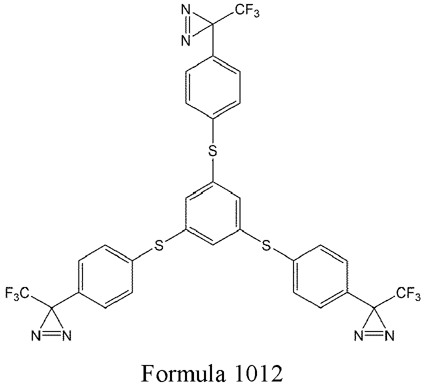

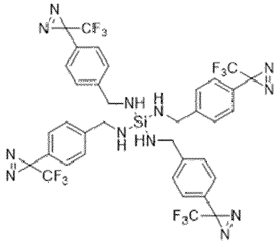

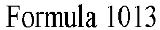

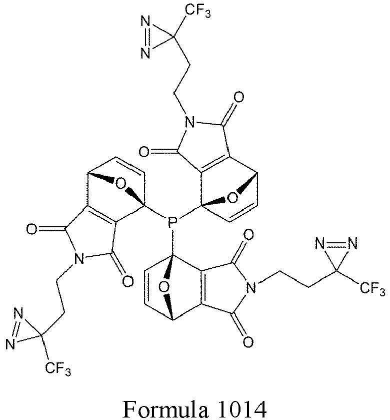

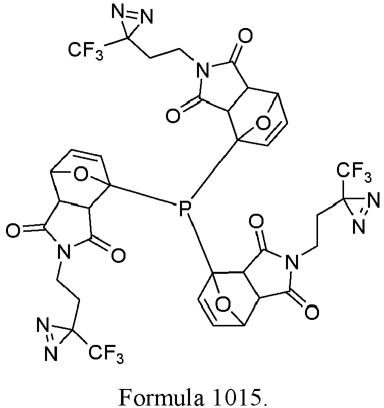

The disclosure also provides an adhesive comprising a compound described herein.

The disclosure also provides a resin mixture comprising a first polymer precursor comprising a compound described herein, and a second polymer precursor comprising a different compound comprising a polymerizable or crosslinkable group. In some embodiments, the resin mixture further comprises a third polymer precursor comprising a different compound comprising a polymerizable or crosslinkable group. In some embodiments, the second polymer precursor is partially or totally polymerized or crosslinked. In some embodiments, the first polymer precursor is partially or totally polymerized or crosslinked. In some embodiments, the third polymer precursor is partially or totally polymerized or crosslinked. In some embodiments, the mixture comprises an elastomer.

The disclosure also provides a method of making a compound described herein, the method comprising reacting a first precursor comprising a diazirine group and a nucleophilic group with a second precursor comprising a leaving group.

The disclosure also provides a method of bonding two or more substrates, comprising contacting the substrates with a compound described herein, with an adhesive described herein, or with a resin mixture described herein. In some embodiments, at least one substrate comprises an elastomer. In some embodiments, a method of bonding two or more substrates further comprises exposure to an actinic radiation. In some embodiments, the actinic radiation is patterned to bond selected interfacial areas between the substrates. In some embodiments, a method of bonding two or more substrates further comprises exposure to an elevated temperature. In some embodiments, an elevated temperature is any temperature above room temperature and below the lowest decomposition temperature of any one of a substrate described herein, a compound described herein, an adhesive described herein, or a resin mixture described herein. In some embodiments, a method of bonding two or more substrates further comprises washing a portion of an unreacted compound described herein. In some embodiments, a substrate comprises a silicone. In some embodiments, a substrate comprises polydimethylsiloxane. In some embodiments, a substrate is comprised in a fluidic elastomer actuator. In some embodiments, a substrate comprises a polymer comprising at least one aliphatic moiety. In some embodiments, a substrate described herein comprises one or more of polyester, polyamide, polyaramid, polytetrafluoroethylene, polyethylene, polypropylene, polyurethane, silicone, polyethyleneglycol, polystyrene, polyethylene terephthalate, nylon, and LYCRA. In some embodiments, a substrate is comprised in a clothing garment. In some embodiments, the clothing garment is a glove.

### DETAILED DESCRIPTION OF EXAMPLES OF THE INVENTION

The disclosure relates generally to multifunctional diazirine bonding of elastomers. Diazirines and/or methods of use thereof, including methods for crosslinking polymers, are known in the art, e.g., US 20160083352, US 20180186747, and WO2020215144. Diazirines are also described by LePage et al., "A broadly applicable crosslinker for aliphatic polymers containing C-H bonds," Science 366, 875-878 (2019).

### Definitions

Unless defined otherwise, all technical and scientific terms used herein have the same meaning as is commonly understood by one of skill in the art to which this disclosure belongs. All patents and publications referred to herein are incorporated by reference in their entireties.

When ranges are used herein to describe, for example, physical or chemical properties such as molecular weight or chemical formulae, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, or from 0% to 10%, or from 0% to 5% of the stated number or numerical range. The term "including" (and related terms such as "comprise" or "comprises" or "having" or "including") includes those embodiments such as, for example, an embodiment of any composition of matter, method or process that "consist of' or "consist essentially of' the described features.

As used herein, the term "cohesive failure" refers to the strength of the adhesive exceeding that of the substrates. Failure mode is fracture of the substrate.

As used herein, the term "adhesive failure" refers to the strength of the adhesive being less than that of the substrates. Failure mode is fracture of the adhesive.

As used herein, the term "multifunctional diazirine" refers to a molecule containing two or more diazirine units.

As used herein, the term "self-bonding diazirine" refers to a molecule containing at least one diazirine unit and at least one sterically aliphatic C-H group. Conjugated carbon rings and CF₃ groups are often employed to create diazirine molecules that do not self-bond.

As used herein, the term "volatile compound" refers to any chemical with a high vapor pressure and/or a boiling point below about 150° C. Examples of volatile compounds include: acetone, methylene chloride, toluene, etc. An article, mixture or component is "volatile compound free" if the article, mixture or component does not include a volatile compound.

As used herein, the term "oligomer" refers to a polymer having a limited number of repeating units, for example, but without limitation, approximately 30 repeat units or less, or any large molecule able to diffuse at least about 100 nm in approximately 2 minutes at room temperature when dissolved in an article of the present disclosure. Such oligomers may contain one or more polymerizable groups whereby the polymerizable groups may be the same or different from other possible monomers in the polymerizable component. Furthermore, when more than one polymerizable group is present on the oligomer, they may be the same or different. Additionally, oligomers may be dendritic. Oligomers are considered herein to be photoactive monomers, although they are sometimes referred to as "photoactive oligomer(s)".

As used herein, the term "free radical polymerization" refers to any polymerization reaction that is initiated by any molecule comprising a free radical or radicals.

As used herein, the term "cationic polymerization" refers to any polymerization reaction that is initiated by any molecule comprising a cationic moiety or moieties.

As used herein, the term "anionic polymerization" refers to any polymerization reaction that is initiated by any molecule comprising an anionic moiety or moieties.

As used herein, the term "photoinitiator" refers to the conventional meaning of the term photoinitiator and also refers to sensitizers and dyes. In general, a photoinitiator causes the light initiated polymerization of a material, such as a photoactive oligomer or monomer, when the material containing the photoinitiator is exposed to light of a wavelength that activates the photoinitiator, e.g., a photoinitiating light source. The photoinitiator may refer to a combination of components, some of which individually are not light sensitive, yet in combination are capable of curing the photoactive oligomer or monomer, examples of which include a dye/amine, a sensitizer/iodonium salt, a dye/borate salt, etc.

As used herein, the term "photoinitiator component" refers to a single photoinitiator or a combination of two or more photoinitiators. For example, two or more photoinitiators may be used in the photoinitiator component of the present disclosure to allow recording at two or more different wavelengths of light.

As used herein, the term "polymerizable component" refers to one or more photoactive polymerizable materials, and possibly one or more additional polymerizable materials, e.g., monomers and/or oligomers, that are capable of forming a polymer.

As used herein, the term "polymerizable moiety" refers to a chemical group capable of participating in a polymerization reaction, at any level, for example, initiation, propagation, etc. Polymerizable moieties include, but are not limited to, addition polymerizable moieties and condensation polymerizable moieties. Polymerizable moieties include, but are not limited to, double bonds, triple bonds, and the like.

As used herein, the term "photoactive polymerizable material" refers to a monomer, an oligomer and combinations thereof that polymerize in the presence of a photoinitiator that has been activated by being exposed to a photoinitiating light source, e.g., recording light. In reference to the functional group that undergoes curing, the photoactive polymerizable material comprises at least one such functional group. It is also understood that there exist photoactive polymerizable materials that are also photoinitiators, such as N-methylmaleimide, derivatized acetophenones, etc., and that in such a case, it is understood that the photoactive monomer and/or oligomer of the present disclosure may also be a photoinitiator.

As used herein, the term "photopolymer" refers to a polymer formed by one or more photoactive polymerizable materials, and possibly one or more additional monomers and/or oligomers.

As used herein, the term "polymerization retarder" refers to one or more compositions, compounds, molecules, etc., that are capable of slowing, reducing, etc., the rate of polymerization while the photoinitiating light source is off or absent, or inhibiting the polymerization of the polymerizable component when the photoinitiating light source is off or absent. A polymerization retarder is typically slow to react with a radical (compared to an inhibitor), thus while the photoinitiating light source is on, polymerization continues at a reduced rate because some of the radicals are effectively terminated by the retarder. In some embodiments, at high enough concentrations, a polymerization retarder can potentially behave as a polymerization inhibitor. In some embodiments, it is desirable to be within the concentration range that allows for retardation of polymerization to occur, rather than inhibition of polymerization.

As used herein, the term "polymerization inhibitor" refers to one or more compositions, compounds, molecules, etc., that are capable of inhibiting or substantially inhibiting the polymerization of the polymerizable component when the photoinitiating light source is on or off. Polymerization inhibitors typically react very quickly with radicals and effectively stop a polymerization reaction. Inhibitors cause an inhibition time during which little to no photopolymer forms, e.g., only very small chains. Typically, photopolymerization occurs only after nearly 100% of the inhibitor is reacted.

As used herein, the term "chain transfer agent" refers to one or more compositions, compounds, molecules, etc. that are capable of interrupting the growth of a polymeric molecular chain by formation of a new radical that may react as a new nucleus for forming a new polymeric molecular chain. Typically, chain transfer agents cause the formation of a higher proportion of shorter polymer chains, relative to polymerization reactions that occur in the absence of chain transfer agents. In some embodiments, certain chain transfer agents can behave as retarders or inhibitors if they do not efficiently reinitiate polymerization.

As used herein, the term "metastable reactive centers" refers to one or more compositions, compounds, molecules, etc., that have the ability to create pseudo-living radical polymerizations with certain polymerizable components. It is also understood that infrared light or heat may be used to activate metastable reactive centers towards polymerization.

As used herein, the term "light or heat labile phototerminators" refers to one or more compositions, compounds, components, materials, molecules, etc., capable of undergoing reversible termination reactions using a light source and/or heat.

As used herein, the terms "photo-acid generators," "photo-base generators," and "photogenerated radicals," refer to one or more compositions, compounds, molecules, etc., that, when exposed to a light source, generate one or more compositions, compounds, molecules, etc., that are acidic, basic, or a free radical.

As used herein, the term "polarity or solvation effects" refers to an effect or effects that the solvent or the polarity of the medium has on the polymerization rate. This effect is most pronounced for ionic polymerizations where the proximity of the counter ion to the reactive chain end influences the polymerization rate.

As used herein, the term "counter ion effects" refers to the effect that counter ion, in ionic polymerizations, has on the kinetic chain length. Good counter ions allow for very long kinetic chain lengths, whereas poor counter ions tend to collapse with the reactive chain end, thus terminating the kinetic chain (e.g., causing smaller chains to be formed).

As used herein, the term "plasticizer" refers to the conventional meaning of the term plasticizer. In general, a plasticizer is a compound added to a polymer both to facilitate processing and to increase the flexibility and/or toughness of a product by internal modification (solvation) of a polymer molecule.

As used herein, the term "thermoplastic" refers to the conventional meaning of thermoplastic, e.g., a composition, compound, substance, etc., that exhibits the property of a material, such as a high polymer, that softens when exposed to heat and generally returns to its original condition when cooled to room temperature. Examples of thermoplastics include, but are not limited to: poly(methyl vinyl ether-alt-maleic anhydride), poly(vinyl acetate), poly(styrene), poly(propylene), poly(ethylene oxide), linear nylons, linear polyesters, linear polycarbonates, linear polyurethanes, etc.

As used herein, the term "room temperature thermoplastic" refers to a thermoplastic that is solid at room temperature, e.g., will not cold flow at room temperature.

As used herein, the term "room temperature" refers to the commonly accepted meaning of room temperature.

As used herein, the term "thermoset" refers to the conventional meaning of thermoset, e.g., a composition, compound, substance, etc., that is crosslinked such that it does not have a melting temperature. Examples of thermosets are crosslinked poly(urethanes), crosslinked poly(acrylates), crosslinked poly(styrene), etc.

Unless otherwise stated, the chemical structures depicted herein are intended to include compounds which differ only in the presence of one or more isotopically enriched atoms. For example, compounds where one or more hydrogen atoms is replaced by deuterium or tritium, or where one or more carbon atoms is replaced by ¹³C- or ¹⁴C-enriched carbons, are within the scope of this disclosure.

"Alkyl" refers to a straight or branched hydrocarbon chain radical consisting solely of carbon and hydrogen atoms, containing no unsaturation, having from one to ten carbon atoms (*e*.*g*., (Ci-io)alkyl or C₁-₁₀ alkyl). Whenever it appears herein, a numerical range such as "1 to 10" refers to each integer in the given range - *e.g.,* "1 to 10 carbon atoms" means that the alkyl group may consist of 1 carbon atom, 2 carbon atoms, 3 carbon atoms, *etc.,* up to and including 10 carbon atoms, although the definition is also intended to cover the occurrence of the term "alkyl" where no numerical range is specifically designated. Typical alkyl groups include, but are in no way limited to, methyl, ethyl, propyl, isopropyl, *n*-butyl, isobutyl, *sec-*butyl isobutyl, tertiary butyl, pentyl, isopentyl, neopentyl, hexyl, heptyl, octyl, nonyl and decyl. The alkyl moiety may be attached to the rest of the molecule by a single bond, such as for example, methyl (Me), ethyl (Et), *n*-propyl (Pr), 1-methylethyl (isopropyl), *n*-butyl, *n-*pentyl, 1,1-dimethylethyl (*t*-butyl) and 3-methylhexyl. Unless stated otherwise specifically in the specification, an alkyl group is optionally substituted by one or more of substituents which are independently heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, - C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), - S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂ where each R^{a} is independently hydrogen, fluoroalkyl, carbocyclyl, carbocyclylalkyl, aryl, aralkyl, heterocycloalkyl, heterocycloalkylalkyl, heteroaryl or heteroarylalkyl.

"Alkylaryl" refers to an -(alkyl)aryl radical where aryl and alkyl are as disclosed herein and which are optionally substituted by one or more of the substituents described as suitable substituents for aryl and alkyl respectively.

"Alkylhetaryl" refers to an -(alkyl)hetaryl radical where hetaryl and alkyl are as disclosed herein and which are optionally substituted by one or more of the substituents described as suitable substituents for aryl and alkyl respectively.

"Alkylheterocycloalkyl" refers to an -(alkyl) heterocyclyl radical where alkyl and heterocycloalkyl are as disclosed herein and which are optionally substituted by one or more of the substituents described as suitable substituents for heterocycloalkyl and alkyl respectively.

An "alkene" moiety refers to a group consisting of at least two carbon atoms and at least one carbon-carbon double bond, and an "alkyne" moiety refers to a group consisting of at least two carbon atoms and at least one carbon-carbon triple bond. The alkyl moiety, whether saturated or unsaturated, may be branched, straight chain, or cyclic.

"Alkenyl" refers to a straight or branched hydrocarbon chain radical group consisting solely of carbon and hydrogen atoms, containing at least one double bond, and having from two to ten carbon atoms (*e*.*g*., (C₂-₁₀)alkenyl or C₂-₁₀ alkenyl). Whenever it appears herein, a numerical range such as "2 to 10" refers to each integer in the given range - *e.g.,* "2 to 10 carbon atoms" means that the alkenyl group may consist of 2 carbon atoms, 3 carbon atoms, *etc.,* up to and including 10 carbon atoms. The alkenyl moiety may be attached to the rest of the molecule by a single bond, such as for example, ethenyl (*e.g*., vinyl), prop-1-enyl (*e.g*., allyl), but-1-enyl, pent-1-enyl and penta-1,4-dienyl. Unless stated otherwise specifically in the specification, an alkenyl group is optionally substituted by one or more substituents which are independently alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, - C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), - S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Alkenyl-cycloalkyl" refers to an -(alkenyl)cycloalkyl radical where alkenyl and cycloalkyl are as disclosed herein and which are optionally substituted by one or more of the substituents described as suitable substituents for alkenyl and cycloalkyl respectively.

"Alkynyl" refers to a straight or branched hydrocarbon chain radical group consisting solely of carbon and hydrogen atoms, containing at least one triple bond, having from two to ten carbon atoms (*e*.*g*., (C₂-₁₀)alkynyl or C₂-₁₀ alkynyl). Whenever it appears herein, a numerical range such as "2 to 10" refers to each integer in the given range - *e.g.,* "2 to 10 carbon atoms" means that the alkynyl group may consist of 2 carbon atoms, 3 carbon atoms, *etc.,* up to and including 10 carbon atoms. The alkynyl may be attached to the rest of the molecule by a single bond, for example, ethynyl, propynyl, butynyl, pentynyl and hexynyl. Unless stated otherwise specifically in the specification, an alkynyl group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), - S(O)ₜR^{a} (where t is 1 or 2), -S(O)ₜOR^{a} (where t is 1 or 2), -S(O)ₜN(R^{a})₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Alkynyl-cycloalkyl" refers to an -(alkynyl)cycloalkyl radical where alkynyl and cycloalkyl are as disclosed herein and which are optionally substituted by one or more of the substituents described as suitable substituents for alkynyl and cycloalkyl respectively.

"Carboxaldehyde" refers to a -(C=O)H radical.

"Carboxyl" refers to a -(C=O)OH radical.

"Cyano" refers to a -CN radical.

"Cycloalkyl" refers to a monocyclic or polycyclic radical that contains only carbon and hydrogen, and may be saturated, or partially unsaturated. Cycloalkyl groups include groups having from 3 to 10 ring atoms (*e*.*g*. (C₃-₁₀)cycloalkyl or C₃-₁₀ cycloalkyl). Whenever it appears herein, a numerical range such as "3 to 10" refers to each integer in the given range - *e.g.,* "3 to 10 carbon atoms" means that the cycloalkyl group may consist of 3 carbon atoms, *etc.,* up to and including 10 carbon atoms. Illustrative examples of cycloalkyl groups include, but are not limited to the following moieties: cyclopropyl, cyclobutyl, cyclopentyl, cyclopentenyl, cyclohexyl, cyclohexenyl, cycloheptyl, cyclooctyl, cyclononyl, cyclodecyl, norbornyl, and the like. Unless stated otherwise specifically in the specification, a cycloalkyl group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, - OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a} (where t is 1 or 2), -S(O)ₜR^{a} (where t is 1 or 2), - S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Cycloalkyl-alkenyl" refers to a -(cycloalkyl)alkenyl radical where cycloalkyl and alkenyl are as disclosed herein and which are optionally substituted by one or more of the substituents described as suitable substituents for cycloalkyl and alkenyl, respectively.

"Cycloalkyl-heterocycloalkyl" refers to a -(cycloalkyl)heterocycloalkyl radical where cycloalkyl and heterocycloalkyl are as disclosed herein and which are optionally substituted by one or more of the substituents described as suitable substituents for cycloalkyl and heterocycloalkyl, respectively.

"Cycloalkyl-heteroaryl" refers to a -(cycloalkyl)heteroaryl radical where cycloalkyl and heteroaryl are as disclosed herein and which are optionally substituted by one or more of the substituents described as suitable substituents for cycloalkyl and heteroaryl, respectively.

The term "alkoxy" refers to the group -O-alkyl, including from 1 to 8 carbon atoms of a straight, branched, cyclic configuration and combinations thereof attached to the parent structure through an oxygen. Examples include, but are not limited to, methoxy, ethoxy, propoxy, isopropoxy, cyclopropyloxy and cyclohexyloxy. "Lower alkoxy" refers to alkoxy groups containing one to six carbons.

The term "substituted alkoxy" refers to alkoxy where the alkyl constituent is substituted (*e*.*g*., -O-(substituted alkyl)). Unless stated otherwise specifically in the specification, the alkyl moiety of an alkoxy group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, - N(R^{a})₂, -C(O)R^{a}, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), - S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

The term "alkoxycarbonyl" refers to a group of the formula (alkoxy)(C=O)- attached through the carbonyl carbon where the alkoxy group has the indicated number of carbon atoms. Thus a (C₁-₆)alkoxycarbonyl group is an alkoxy group having from 1 to 6 carbon atoms attached through its oxygen to a carbonyl linker. "Lower alkoxycarbonyl" refers to an alkoxycarbonyl group where the alkoxy group is a lower alkoxy group.

The term "substituted alkoxycarbonyl" refers to the group (substituted alkyl)-O-C(O)-where the group is attached to the parent structure through the carbonyl functionality. Unless stated otherwise specifically in the specification, the alkyl moiety of an alkoxycarbonyl group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -C(O)SR^{a}, -SC(O)R^{a},-OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a} (where t is 1 or 2), -S(O)ₜR^{a} (where t is 1 or 2), - S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Acyl" refers to the groups (alkyl)-C(O)-, (aryl)-C(O)-, (heteroaryl)-C(O)-, (heteroalkyl)-C(O)- and (heterocycloalkyl)-C(O)-, where the group is attached to the parent structure through the carbonyl functionality. If the R radical is heteroaryl or heterocycloalkyl, the hetero ring or chain atoms contribute to the total number of chain or ring atoms. Unless stated otherwise specifically in the specification, the alkyl, aryl or heteroaryl moiety of the acyl group is optionally substituted by one or more substituents which are independently alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -C(O)SR^{a}, -SC(O)R^{a},-OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a} (where t is 1 or 2), -S(O)ₜR^{a} (where t is 1 or 2), - S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Acyloxy" refers to a R(C=O)O- radical where R is alkyl, aryl, heteroaryl, heteroalkyl or heterocycloalkyl, which are as described herein. If the R radical is heteroaryl or heterocycloalkyl, the hetero ring or chain atoms contribute to the total number of chain or ring atoms. Unless stated otherwise specifically in the specification, the R of an acyloxy group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -C(O)SR^{a}, -SC(O)R^{a},-OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a} (where t is 1 or 2), -S(O)ₜR^{a} (where t is 1 or 2), - S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Amino" or "amine" refers to a -N(R^{a})₂ radical group, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl, unless stated otherwise specifically in the specification. When a -N(R^{a})₂ group has two R^{a} substituents other than hydrogen, they can be combined with the nitrogen atom to form a 4-, 5-, 6- or 7-membered ring. For example, -N(R^{a})₂ is intended to include, but is not limited to, 1-pyrrolidinyl and 4-morpholinyl. Unless stated otherwise specifically in the specification, an amino group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -C(O)SR^{a}, -SC(O)R^{a},-OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a} (where t is 1 or 2), -S(O)ₜR^{a} (where t is 1 or 2), - S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

The term "substituted amino" also refers to N-oxides of the groups -NHR^{d}, and - NR^{d}R^{d} each as described above. N-oxides can be prepared by treatment of the corresponding amino group with, for example, hydrogen peroxide or m-chloroperoxybenzoic acid.

"Amide" or "amido" refers to a chemical moiety with formula -C(O)N(R)₂ or -NHC(O)R, where R is selected from the group consisting of hydrogen, alkyl, cycloalkyl, aryl, heteroaryl (bonded through a ring carbon) and heteroalicyclic (bonded through a ring carbon), each of which moiety may itself be optionally substituted. The R₂ of -N(R)₂ of the amide may optionally be taken together with the nitrogen to which it is attached to form a 4-, 5-, 6- or 7-membered ring. Unless stated otherwise specifically in the specification, an amido group is optionally substituted independently by one or more of the substituents as described herein for alkyl, cycloalkyl, aryl, heteroaryl, or heterocycloalkyl. An amide may be an amino acid or a peptide molecule attached to a compound disclosed herein, thereby forming a prodrug. The procedures and specific groups to make such amides are known to those of skill in the art and can readily be found in seminal sources such as Greene and Wuts, Protective Groups in Organic Synthesis, 3rd Ed., John Wiley & Sons, New York, N.Y., 1999, which is incorporated herein by reference in its entirety.

"Aromatic" or "aryl" refers to an aromatic radical with six to ten ring atoms (*e.g.*, C₆-C₁₀ aromatic or C₆-C₁₀ aryl) which has at least one ring having a conjugated pi electron system which is carbocyclic (*e*.*g*., phenyl, fluorenyl, and naphthyl). Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by adding "-idene" to the name of the corresponding univalent radical, *e.g.,* a naphthyl group with two points of attachment is termed naphthylidene. Whenever it appears herein, a numerical range such as "6 to 10" refers to each integer in the given range; *e.g.,* "6 to 10 ring atoms" means that the aryl group may consist of 6 ring atoms, 7 ring atoms, *etc.,* up to and including 10 ring atoms. The term includes monocyclic or fused-ring polycyclic (*e*.*g*., rings which share adjacent pairs of ring atoms) groups. Unless stated otherwise specifically in the specification, an aryl moiety is optionally substituted by one or more substituents which are independently alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, - OC(O)-R^{a}, -N(R^{a})₂, -C(O)R^{a}, -C(O)OR^{a}, -C(O)SR^{a}, -SC(O)R^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), -S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl. It is understood that a substituent R attached to an aromatic ring at an unspecified position, (e.g.: 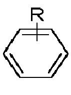 ), includes one or more, and up to the maximum number of possible substituents.

The term "aryloxy" refers to the group -O-aryl.

The term "substituted aryloxy" refers to aryloxy where the aryl substituent is substituted (*e*.*g*., -O-(substituted aryl)). Unless stated otherwise specifically in the specification, the aryl moiety of an aryloxy group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, - N(R^{a})₂, -C(O)R^{a}, -C(O)OR^{a}, -C(O)SR^{a}, -SC(O)R^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), -S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Aralkyl" or "arylalkyl" refers to an (aryl)alkyl-radical where aryl and alkyl are as disclosed herein and which are optionally substituted by one or more of the substituents described as suitable substituents for aryl and alkyl respectively.

"Ester" refers to a chemical radical of formula -COOR, where R is selected from the group consisting of alkyl, cycloalkyl, aryl, heteroaryl (bonded through a ring carbon) and heteroalicyclic (bonded through a ring carbon). The procedures and specific groups to make esters are known to those of skill in the art and can readily be found in seminal sources such as Greene and Wuts, Protective Groups in Organic Synthesis, 3rd Ed., John Wiley & Sons, New York, N.Y., 1999, which is incorporated herein by reference in its entirety. Unless stated otherwise specifically in the specification, an ester group is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, trifluoromethyl, trifluoromethoxy, nitro, trimethylsilanyl, -OR^{a}, -SR^{a}, -OC(O)-R^{a}, -N(R^{a})₂, -C(O)R^{a}, -C(O)OR^{a}, -C(O)SR^{a}, -SC(O)R^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), -S(O)ₜR^{a} (where t is 1 or 2), -S(O)ₜOR^{a} (where t is 1 or 2), -S(O)ₜN(R^{a})₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Fluoroalkyl" refers to an alkyl radical, as defined above, that is substituted by one or more fluoro radicals, as defined above, for example, trifluoromethyl, difluoromethyl, 2,2,2-trifluoroethyl, 1-fluoromethyl-2-fluoroethyl, and the like. The alkyl part of the fluoroalkyl radical may be optionally substituted as defined above for an alkyl group.

"Halo," "halide," or, alternatively, "halogen" is intended to mean fluoro, chloro, bromo or iodo. The terms "haloalkyl," "haloalkenyl," "haloalkynyl," and "haloalkoxy" include alkyl, alkenyl, alkynyl and alkoxy structures that are substituted with one or more halo groups or with combinations thereof. For example, the terms "fluoroalkyl" and "fluoroalkoxy" include haloalkyl and haloalkoxy groups, respectively, in which the halo is fluorine.

"Heteroalkyl," "heteroalkenyl," and "heteroalkynyl" refer to optionally substituted alkyl, alkenyl and alkynyl radicals and which have one or more skeletal chain atoms selected from an atom other than carbon, *e*.*g*., oxygen, nitrogen, sulfur, phosphorus or combinations thereof. A numerical range may be given - *e.g.*, C₁-C₄ heteroalkyl which refers to the chain length in total, which in this example is 4 atoms long. A heteroalkyl group may be substituted with one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, nitro, oxo, thioxo, trimethylsilanyl, -OR^{a}, -SR^{a}, -OC(O)-R^{a}, - N(R^{a})₂, -C(O)R^{a}, -C(O)OR^{a}, -C(O)SR^{a}, -SC(O)R^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), -S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Heteroalkylaryl" refers to an -(heteroalkyl)aryl radical where heteroalkyl and aryl are as disclosed herein and which are optionally substituted by one or more of the substituents described as suitable substituents for heteroalkyl and aryl, respectively.

"Heteroalkylheteroaryl" refers to an -(heteroalkyl)heteroaryl radical where heteroalkyl and heteroaryl are as disclosed herein and which are optionally substituted by one or more of the substituents described as suitable substituents for heteroalkyl and heteroaryl, respectively.

"Heteroalkylheterocycloalkyl" refers to an -(heteroalkyl)heterocycloalkyl radical where heteroalkyl and heterocycloalkyl are as disclosed herein and which are optionally substituted by one or more of the substituents described as suitable substituents for heteroalkyl and heterocycloalkyl, respectively.

"Heteroalkylcycloalkyl" refers to an -(heteroalkyl)cycloalkyl radical where heteroalkyl and cycloalkyl are as disclosed herein and which are optionally substituted by one or more of the substituents described as suitable substituents for heteroalkyl and cycloalkyl, respectively.

"Heteroaryl" or "heteroaromatic" refers to a 5- to 18-membered aromatic radical (*e.g.*, C₅-C₁₃ heteroaryl) that includes one or more ring heteroatoms selected from nitrogen, oxygen and sulfur, and which may be a monocyclic, bicyclic, tricyclic or tetracyclic ring system. Whenever it appears herein, a numerical range such as "5 to 18" refers to each integer in the given range - *e.g.,* "5 to 18 ring atoms" means that the heteroaryl group may consist of 5 ring atoms, 6 ring atoms, etc., up to and including 18 ring atoms. Bivalent radicals derived from univalent heteroaryl radicals whose names end in "-yl" by removal of one hydrogen atom from the atom with the free valence are named by adding "-idene" to the name of the corresponding univalent radical - *e.g.,* a pyridyl group with two points of attachment is a pyridylidene. A N-containing "heteroaromatic" or "heteroaryl" moiety refers to an aromatic group in which at least one of the skeletal atoms of the ring is a nitrogen atom. The polycyclic heteroaryl group may be fused or non-fused. The heteroatom(s) in the heteroaryl radical are optionally oxidized. One or more nitrogen atoms, if present, are optionally quaternized. The heteroaryl may be attached to the rest of the molecule through any atom of the ring(s). Examples of heteroaryls include, but are not limited to, azepinyl, acridinyl, benzimidazolyl, benzindolyl, 1,3-benzodioxolyl, benzofuranyl, benzooxazolyl, benzo[*d*]thiazolyl, benzothiadiazolyl, benzo[*b*][1,4]dioxepinyl, benzo[*b*][1,4]oxazinyl, 1,4-benzodioxanyl, benzonaphthofuranyl, benzoxazolyl, benzodioxolyl, benzodioxinyl, benzoxazolyl, benzopyranyl, benzopyranonyl, benzofuranyl, benzofuranonyl, benzofurazanyl, benzothiazolyl, benzothienyl(benzothiophenyl), benzothieno[3,2-*d*]pyrimidinyl, benzotriazolyl, benzo[4,6]imidazo[1,2-*a*]pyridinyl, carbazolyl, cinnolinyl, cyclopenta[*d*]pyrimidinyl, 6,7-dihydro-5*H*-cyclopenta[4,5]thieno[2,3-*d*]pyrimidinyl, 5,6-dihydrobenzo[*h*]quinazolinyl, 5,6-dihydrobenzo[*h*]cinnolinyl, 6,7-dihydro-5*H-*benzo[6,7]cyclohepta[1,2-*c*]pyridazinyl, dibenzofuranyl, dibenzothiophenyl, furanyl, furazanyl, furanonyl, furo[3,2-*c*]pyridinyl, 5,6,7,8,9,10-hexahydrocycloocta[*d*]pyrimidinyl, 5,6,7,8,9,10-hexahydrocycloocta[*d*]pyridazinyl, 5,6,7,8,9,10-hexahydrocycloocta[*d*]pyridinyl, isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[*h*]quinazolinyl, 1-phenyl-1*H*-pyrrolyl, phenazinyl, phenothiazinyl, phenoxazinyl, phthalazinyl, pteridinyl, purinyl, pyranyl, pyrrolyl, pyrazolyl, pyrazolo[3,4-d]pyrimidinyl, pyridinyl, pyrido[3,2-*d*]pyrimidinyl, pyrido[3,4-*d*]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5*H-*cyclohepta[4,5]thieno[2,3-*d*]pyrimidinyl, 5,6,7,8-tetrahydropyrido[4,5-*c*]pyridazinyl, thiazolyl, thiadiazolyl, thiapyranyl, triazolyl, tetrazolyl, triazinyl, thieno[2,3-*d*]pyrimidinyl, thieno[3,2-*d*]pyrimidinyl, thieno[2,3-c]pyridinyl, and thiophenyl (*e*.*g*., thienyl). Unless stated otherwise specifically in the specification, a heteroaryl moiety is optionally substituted by one or more substituents which are independently: alkyl, heteroalkyl, alkenyl, alkynyl, cycloalkyl, heterocycloalkyl, aryl, arylalkyl, heteroaryl, heteroarylalkyl, hydroxy, halo, cyano, nitro, oxo, thioxo, trimethylsilanyl, -OR^{a}, -SR^{a}, -OC(O)-R^{a}, - N(R^{a})₂, -C(O)R^{a}, -C(O)OR^{a}, -C(O)SR^{a}, -SC(O)R^{a}, -OC(O)N(R^{a})₂, -C(O)N(R^{a})₂, - N(R^{a})C(O)OR^{a}, -N(R^{a})C(O)R^{a}, -N(R^{a})C(O)N(R^{a})₂, -N(R^{a})C(NR^{a})N(R^{a})₂, -N(R^{a})S(O)ₜR^{a} (where t is 1 or 2), -S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

Substituted heteroaryl also includes ring systems substituted with one or more oxide (-O-) substituents, such as, for example, pyridinyl N-oxides.

"Heteroarylalkyl" refers to a moiety having an aryl moiety, as described herein, connected to an alkylene moiety, as described herein, where the connection to the remainder of the molecule is through the alkylene group.

"Heterocycloalkyl" refers to a stable 3- to 18-membered non-aromatic ring radical that comprises two to twelve carbon atoms and from one to six heteroatoms selected from nitrogen, oxygen and sulfur. Whenever it appears herein, a numerical range such as "3 to 18" refers to each integer in the given range - *e.g.,* "3 to 18 ring atoms" means that the heterocycloalkyl group may consist of 3 ring atoms, 4 ring atoms, *etc.,* up to and including 18 ring atoms. Unless stated otherwise specifically in the specification, the heterocycloalkyl radical is a monocyclic, bicyclic, tricyclic or tetracyclic ring system, which may include fused or bridged ring systems. The heteroatoms in the heterocycloalkyl radical may be optionally oxidized. One or more nitrogen atoms, if present, are optionally quaternized. The heterocycloalkyl radical is partially or fully saturated. The heterocycloalkyl may be attached to the rest of the molecule through any atom of the ring(s). Examples of such heterocycloalkyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, a heterocycloalkyl moiety is optionally substituted by one or more substituents which independently are: alkyl, heteroalkyl, alkenyl, alkynyl, cycloalkyl, heterocycloalkyl, aryl, arylalkyl, heteroaryl, heteroarylalkyl, hydroxy, halo, cyano, nitro, oxo, thioxo, trimethylsilanyl, -OR^{a}, - SR^{a}, -OC(O)-R^{a}, -N(R^{a})₂, -C(O)R^{a}, -C(O)OR^{a}, -C(O)SR^{a}, -SC(O)R^{a}, -OC(O)N(R^{a})₂, - C(O)N(R^{a})₂, -N(R^{a})C(O)OR^{a}, -N(R^{a})C(O)R^{a}, -N(R^{a})C(O)N(R^{a})₂, -N(R^{a})C(NR^{a})N(R^{a})₂, - N(R^{a})S(O)ₜR^{a} (where t is 1 or 2), -S(O)ₜR^{a} (where t is 1 or 2), -S(O)ₜOR^{a} (where t is 1 or 2), -S(O)ₜN(R)₂ (where t is 1 or 2), -S(O)ₜN(R^{a})C(O)R^{a} (where t is 1 or 2), or PO₃(R^{a})₂, where each R^{a} is independently hydrogen, alkyl, fluoroalkyl, carbocyclyl, carbocyclylalkyl, aryl, aralkyl, heterocycloalkyl, heterocycloalkylalkyl, heteroaryl or heteroarylalkyl.

"Heterocycloalkyl" also includes bicyclic ring systems where one non-aromatic ring, usually with 3 to 7 ring atoms, contains at least 2 carbon atoms in addition to 1-3 heteroatoms independently selected from oxygen, sulfur, and nitrogen, as well as combinations including at least one of the foregoing heteroatoms; and the other ring, usually with 3 to 7 ring atoms, optionally contains 1-3 heteroatoms independently selected from oxygen, sulfur, and nitrogen and is not aromatic.

"Nitro" refers to the -NO₂ radical.

"Oxa" refers to the -O- radical.

"Oxo" refers to the =O radical.

"Isomers" are different compounds that have the same molecular formula. "Stereoisomers" are isomers that differ only in the way the atoms are arranged in space - *e.g.*, having a different stereochemical configuration. "Enantiomers" are a pair of stereoisomers that are non-superimposable mirror images of each other. A 1:1 mixture of a pair of enantiomers is a "racemic" mixture. The term "(±)" is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R-S system. When a compound is a pure enantiomer the stereochemistry at each chiral carbon can be specified by either (*R*) or (*S*). Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and can thus give rise to enantiomers, diastereomers, and other stereoisomeric forms that can be defined, in terms of absolute stereochemistry, as (*R*) or (*S*). The present chemical entities, compositions and methods are meant to include all such possible isomers, including racemic mixtures, optically pure forms and intermediate mixtures. Optically active (*R*)- and (*S*)-isomers can be prepared using chiral synthons or chiral reagents, or resolved using conventional techniques. When the compounds described herein contain olefinic double bonds or other centers of geometric asymmetry, and unless specified otherwise, it is intended that the compounds include both *E* and *Z* geometric isomers.

"Enantiomeric purity" as used herein refers to the relative amounts, expressed as a percentage, of the presence of a specific enantiomer relative to the other enantiomer. For example, if a compound, which may potentially have an (*R*)- or an (*S*)-isomeric configuration, is present as a racemic mixture, the enantiomeric purity is about 50% with respect to either the (*R*)- or (*S*)-isomer. If that compound has one isomeric form predominant over the other, for example, 80% (*S*)-isomer and 20% (*R*)-isomer, the enantiomeric purity of the compound with respect to the (*S*)-isomeric form is 80%. The enantiomeric purity of a compound can be determined in a number of ways known in the art, including but not limited to chromatography using a chiral support, polarimetric measurement of the rotation of polarized light, nuclear magnetic resonance spectroscopy using chiral shift reagents which include but are not limited to lanthanide containing chiral complexes or Pirkle's reagents, or derivatization of a compounds using a chiral compound such as Mosher's acid followed by chromatography or nuclear magnetic resonance spectroscopy.

In some embodiments, enantiomerically enriched compositions have different properties than the racemic mixture of that composition. Enantiomers can be isolated from mixtures by methods known to those skilled in the art, including chiral high pressure liquid chromatography (HPLC) and the formation and crystallization of chiral salts; or preferred enantiomers can be prepared by asymmetric syntheses. See, for example, Jacques, et al., Enantiomers, Racemates and Resolutions, Wiley Interscience, New York (1981); E. L. Eliel, Stereochemistry of Carbon Compounds, McGraw-Hill, New York (1962); and E. L. Eliel and S. H. Wilen, Stereochemistry of Organic Compounds, Wiley-Interscience, New York (1994).

The terms "enantiomerically enriched" and "non-racemic," as used herein, refer to compositions in which the percent by weight of one enantiomer is greater than the amount of that one enantiomer in a control mixture of the racemic composition (*e*.*g*., greater than 1:1 by weight). For example, an enantiomerically enriched preparation of the (*S*)-enantiomer, means a preparation of the compound having greater than 50% by weight of the (*S*)-enantiomer relative to the (*R*)-enantiomer, such as at least 75% by weight, or such as at least 80% by weight. In some embodiments, the enrichment can be significantly greater than 80% by weight, providing a "substantially enantiomerically enriched" or a "substantially non-racemic" preparation, which refers to preparations of compositions which have at least 85% by weight of one enantiomer relative to other enantiomer, such as at least 90% by weight, or such as at least 95% by weight. The terms "enantiomerically pure" or "substantially enantiomerically pure" refers to a composition that comprises at least 98% of a single enantiomer and less than 2% of the opposite enantiomer.

"Moiety" refers to a specific segment or functional group of a molecule. Chemical moieties are often recognized chemical entities embedded in or appended to a molecule.

"Tautomers" are structurally distinct isomers that interconvert by tautomerization. "Tautomerization" is a form of isomerization and includes prototropic or proton-shift tautomerization, which is considered a subset of acid-base chemistry. "Prototropic tautomerization" or "proton-shift tautomerization" involves the migration of a proton accompanied by changes in bond order, often the interchange of a single bond with an adjacent double bond. Where tautomerization is possible (*e*.*g*., in solution), a chemical equilibrium of tautomers can be reached. An example of tautomerization is keto-enol tautomerization. A specific example of keto-enol tautomerization is the interconversion of pentane-2,4-dione and 4-hydroxypent-3-en-2-one tautomers. Another example of tautomerization is phenol-keto tautomerization. A specific example of phenol-keto tautomerization is the interconversion of pyridin-4-ol and pyridin-4(1*H*)-one tautomers.

A "leaving group or atom" is any group or atom that will, under selected reaction conditions, cleave from the starting material, thus promoting reaction at a specified site. Examples of such groups, unless otherwise specified, include halogen atoms and mesyloxy, p-nitrobenzensulphonyloxy and tosyloxy groups.

"Protecting group" is intended to mean a group that selectively blocks one or more reactive sites in a multifunctional compound such that a chemical reaction can be carried out selectively on another unprotected reactive site and the group can then be readily removed or deprotected after the selective reaction is complete. A variety of protecting groups are disclosed, for example, in T. H. Greene and P. G. M. Wuts, Protective Groups in Organic Synthesis, Third Edition, John Wiley & Sons, New York (1999).

"Solvate" refers to a compound in physical association with one or more molecules of a pharmaceutically acceptable solvent.

"Substituted" means that the referenced group may have attached one or more additional groups, radicals or moieties individually and independently selected from, for example, acyl, alkyl, alkylaryl, cycloalkyl, aralkyl, aryl, carbohydrate, carbonate, heteroaryl, heterocycloalkyl, hydroxy, alkoxy, aryloxy, mercapto, alkylthio, arylthio, cyano, halo, carbonyl, ester, thiocarbonyl, isocyanato, thiocyanato, isothiocyanato, nitro, oxo, perhaloalkyl, perfluoroalkyl, phosphate, silyl, sulfinyl, sulfonyl, sulfonamidyl, sulfoxyl, sulfonate, urea, and amino, including mono- and di-substituted amino groups, and protected derivatives thereof. The substituents themselves may be substituted, for example, a cycloalkyl substituent may itself have a halide substituent at one or more of its ring carbons. The term "optionally substituted" means optional substitution with the specified groups, radicals or moieties.

"Sulfanyl" refers to groups that include -S-(optionally substituted alkyl), -S-(optionally substituted aryl), -S-(optionally substituted heteroaryl) and -S-(optionally substituted heterocycloalkyl).

"Sulfinyl" refers to groups that include -S(O)-H, -S(O)-(optionally substituted alkyl), -S(O)-(optionally substituted amino), -S(O)-(optionally substituted aryl), -S(O)-(optionally substituted heteroaryl) and -S(O)-(optionally substituted heterocycloalkyl).

"Sulfonyl" refers to groups that include -S(O₂)-H, -S(O₂)-(optionally substituted alkyl), -S(O₂)-(optionally substituted amino), -S(O₂)-(optionally substituted aryl), -S(O₂)-(optionally substituted heteroaryl), and -S(O₂)-(optionally substituted heterocycloalkyl).

"Sulfonamidyl" or "sulfonamido" refers to a -S(=O)₂-NRR radical, where each R is selected independently from the group consisting of hydrogen, alkyl, cycloalkyl, aryl, heteroaryl (bonded through a ring carbon) and heteroalicyclic (bonded through a ring carbon). The R groups in -NRR of the -S(=O)₂-NRR radical may be taken together with the nitrogen to which it is attached to form a 4-, 5-, 6- or 7-membered ring. A sulfonamido group is optionally substituted by one or more of the substituents described for alkyl, cycloalkyl, aryl, heteroaryl, respectively.

"Sulfoxyl" refers to a -S(=O)₂OH radical.

"Sulfonate" refers to a -S(=O)₂-OR radical, where R is selected from the group consisting of alkyl, cycloalkyl, aryl, heteroaryl (bonded through a ring carbon) and heteroalicyclic (bonded through a ring carbon). A sulfonate group is optionally substituted on R by one or more of the substituents described for alkyl, cycloalkyl, aryl, heteroaryl, respectively.

Compounds of the present disclosure also include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof. "Crystalline form" and "polymorph" are intended to include all crystalline and amorphous forms of the compound, including, for example, polymorphs, pseudopolymorphs, solvates, hydrates, unsolvated polymorphs (including anhydrates), conformational polymorphs, and amorphous forms, as well as mixtures thereof, unless a particular crystalline or amorphous form is referred to.

For the avoidance of doubt, it is intended herein that particular features (for example integers, characteristics, values, uses, diseases, formulae, compounds or groups) described in conjunction with a particular aspect, embodiment or example of the disclosure are to be understood as applicable to any other aspect, embodiment or example described herein unless incompatible therewith. Thus, such features may be used where appropriate in conjunction with any of the definition, claims or embodiments defined herein. All of the features disclosed in this specification (including any accompanying claims, abstract and drawings), and/or all of the steps of any method or process so disclosed, may be combined in any combination, except combinations where at least some of the features and/or steps are mutually exclusive. The present disclosure is not restricted to any details of any disclosed embodiments. The present disclosure extends to any novel one, or novel combination, of the features disclosed in this specification (including any accompanying claims, abstract and drawings), or to any novel one, or any novel combination, of the steps of any method or process so disclosed.

The creation of interchain crosslinks generally requires that functional groups are already present within the polymer structure. In cases where such functionality is absent, high energy processes (e.g., gamma-irradiation or introduction of free radicals) must be used to abstract hydrogen atoms. Such processes are expensive, non-tunable, and do not work for many industrially-important polymers (e.g., polypropylene) due to, e.g., competing chain-fragmentation processes.

These strategies may not always be appropriate, e.g., when crosslinking an existing polymer material that has desirable properties (mechanical strength, ease of production, low cost, durability, etc.) but which lacks functionality within its chemical structure. This includes many extremely important industrial materials. For example, polyethylene and polypropylene are some of the most important petrochemical-derived polymers, but do not easily lend themselves to chemical crosslinking. Similarly, biomass-derived polymers like polylactic acid and biodegradable polymers such as polycaprolactone often lack any crosslinkable functional groups, even though they contain some measure of functionality within their linear chains.

Existing methods for crosslinking unreactive polymers have a number of disadvantages. For example, crosslinked polyethylene can be produced by peroxide-initiated radical crosslinking. In this method, peroxide additives (e.g., dicumyl peroxide) are physically combined with polyethylene through an extrusion process. The resulting peroxide-impregnated polymer is then heated at high temperatures (typically 200-250 °C) to initiate the formation of radicals, which in turn results in abstraction of hydrogen atoms and eventual crosslinking. The main problem with known radical-based crosslinking methods is the need to break a very strong C-H bond. Fundamentally, the need to generate such high-energy species as alkyl radicals means that little-to-no control is possible using crosslinking methods known in the art. Moreover, the carbon-centered radicals produced following cleavage of these strong C-H bonds are highly reactive and can undergo fragmentation (b-scission) reactions at rates that are competitive with crosslinking. This results in breakage of the polymer chains, and therefore reduces material strength.

Crosslinked polyethylene can also be produced by treatment with either gamma-rays or electron beams. As with radical crosslinking methods, these processes proceed via an initial cleavage of strong C-H bonds, and so suffer many of the disadvantages outlined herein. The polymers produced using gamma-rays may, in some cases, have superior mechanical properties to those generated by peroxide-initiated methods, but the substantial costs associated with this process limits its use to the production of small-scale medical devices. Some of these methods (as well as related processes like silanization) generate intermediate radicals which can undergo b-scission and other undesirable side-reactions. b-Scission is reversible, and so tends not to be a limitation for crosslinked polyethylene, since the polymer chains are held close together, the products of radical fragmentation simply recombine to give the original secondary radical intermediate.

Another issue with radical crosslinking is that the intermediates resulting from b-scission can recombine in a regiochemically different manner, ultimately leading to unexpected branching of the polymer structure. This can lead to a loss of crystallinity, and at the very least is difficult to control.

Similarly, the crosslinking processes described above are not easily tunable. There is no provision for controlling the length or rigidity of the crosslink structure, which could be used to mitigating brittleness.

### Multifunctional Diazirine Synthesis

Molecules containing one diazirine species are known due to their utility in tagging of biological materials. These molecules can be utilized as precursors for synthesizing multifunctional diazirine adhesives. In one example, the primary amine group on {4-[3-(trifluoromethyl)-3H-diazirin-3-yl]phenyl}methanamine can be used in a condensation reaction with hydrolyzed alkoxy groups on tetraethyl orthosilicate (Example 1) at room temperature. Numerous alkoxysilanes are available commercially with varying architecture (bipodal, polymeric, etc.), functionality, and chemical nature of the alkoxy group (propoxy, alkoxy, methoxy, ethoxy, etc.). Appropriate selection of functional alkoxy silane as well as the stoichiometric ratio between the alkoxy silane and amine functionalized diazirine enable control over the functionality of the resulting diazirine adhesive molecule. Not all substituents on a silane need to be alkoxy. Completely reacting 3-glycidyloxypropyl triethoxysilane with amine functionalized diazirine will yield an adhesive with 3 diazirine groups and one epoxy group. This will yield an adhesive that can react both aliphatic (-CH) and epoxy groups on the substrate. Alternatively, dimethyldimethoxy silane would yield an adhesive with 3 diazirine groups and one aliphatic methyl group to enable self-reaction. Off stoichiometric ratios of alkoxy silane and amine functionalized diazirine will yield adhesives that can participate both in aliphatic CH-diazirine reactions and conventional condensation reactions on the remaining alkoxy groups. Proper selection of the starting alkoxysilane can also enable appropriate control over the final chemical structure to yield control over chemical properties (i.e., molecular weight, boiling point, melting point, surface tension, etc.).

Other alternative reaction pathways exist to yield multifunctional diazirines from commercial precursors in simple, scalable reactions. The same amine functionalized diazirine can yield a multifunctional diazirine adhesive by reaction with multifunctional sulfonyl chlorides (Example 2). Another reaction pathway can combine brominated diazirine precursors with multifunctional thiols to yield multifunctional diazirine adhesives (Example 3). Diels-Alder reactions (Example 4) can combine alkene functional diazirine precursors with molecules containing multiple conjugated dienes to form our desired multifunctional diazirine adhesive. Off stoichiometric ratios of any reactants described herein allows for precise control over the functionality of the product.

### Multifunctional Diazirine Bonding of Polymers, in Particular Elastomers

In some embodiments, a liquid solution containing a multifunctional diazirine molecule (either a pure multifunctional diazirine molecule heated above its melting point, or a multifunctional diazirine molecule dissolved in a solvent) is dispensed onto Substrate A. Substrate A is then brought into contact with Substrate B. The combined article is then either exposed to actinic radiation or elevated temperatures to generate a carbene from the diazirine. This carbene group then reacts with an aliphatic C-H bond present on substrate A, substrate B, and/or the diazirine molecule.

In some embodiments, sufficient consumption of the diazirine groups yields a percolated network of covalent bonds between Substrate A, Substrate B, and/or the diazirine adhesive. Alternatively, in some embodiments, an additional reaction step bonds a non-diazirine group (epoxy, acrylate, thiol, etc.) on the adhesive molecule with Substrate A, Substrate B, and/or the diazirine molecule. In some embodiments varying ratios of different multifunctional diazirines can be employed.

Alternatively, in some embodiments, the actinic radiation is patterned in such a way to only bond select interfacial areas between the substrates. Unreacted adhesive molecules are removed via washing to prevent later bonding.

In some embodiments, the multifunctional diazirine molecule is incorporated into the liquid precursors of the elastomer substrate. The elastomer is then crosslinked via other means to yield a polymer network swollen with the adhesive. This polymer substrate is then brought into contact with the partner substrate containing aliphatic groups and subjected to actinic radiation or thermal treatment to crosslink the diazirine and aliphatic C-H groups.

In a non-limiting example, substrate A is a silicone (polydimethylsiloxane) based fluidic elastomer actuator. Substrate B is a clothing garment or glove based on aliphatic polymers (nylon, polyamide, etc.). The dispensed multifunctional diazirine molecule is applied and activated with heat, or actinic radiation to join these two substrates. This process can be repeated to bond numerous actuators to the wearable or stacks of actuators to each other.

In some embodiments, the substituents connecting diazirine moieties can be tuned to provide for desired physical properties of the pre-cure and post-cure adhesive. For example, depending on the alkyl, aryl, or other substitution patterns, the viscosity, thermal stability, and reactivity of the carbene in the pre-cured adhesive will change. The combination of substitution patterns and reaction conversion will also determine the flexibility, thermal resistance, optical properties, and strength of the adhesive layer. For example, aryl rings would decrease flexibility but limit self-reaction of the adhesive. Alkyl substitutions would promote flexibility, but any C-H groups would permit self-bonding of adhesive molecules.

Example 5 contains numerous example of relevant derivatives with different substitution patterns. Some examples are bisdiazirines, but multifunctional derivatives (Example 6) can also be synthesized and used to bond various substrates.

The following clauses describe certain embodiments.

Clause 1. An adhesive compound comprising at least one diazirine group.

Clause 2. An adhesive compound comprising at least two diazirine groups.

Clause 3. The adhesive compound of clause 1 or clause 2, further comprising a CH group.

Clause 4. The adhesive compound of any one of clauses 1 to 3, further comprising at least one Si atom.

Clause 5. The adhesive compound of any one of clauses 1 to 4, further comprising at least one aryl group.

Clause 6. The adhesive compound of any one of clauses 1 to 5, further comprising at least one -S- linker.

Clause 7. The adhesive compound of any one of clauses 1 to 6, further comprising at least one P atom.

Clause 8. The adhesive compound of any one of clauses 1 to 7, further comprising at least one CF₃ group.

Clause 9. A method of making the adhesive compound of any one of clauses 1 to 8, comprising reacting a first precursor comprising a diazirine group and a nucleophilic group with a second precursor comprising a leaving group.

Clause 10. A method of bonding two or more polymeric substrates, comprising contacting the substrates with the adhesive compound of any one of clauses 1 to 8.

Clause 17. A polymeric material comprising a resin mixture comprising a first polymer precursor comprising the compound of clause 1, and a second polymer precursor comprising a different compound comprising a polymerizable or crosslinkable group.

Clause 18. The polymeric material of clause 17, wherein the resin mixture further comprises a third polymer precursor comprising a different compound comprising a polymerizable or crosslinkable group.

Clause 19. The polymeric material of clause 17, wherein the second polymer precursor is partially or totally polymerized or crosslinked.

Clause 20. The polymeric material of clause 17, wherein the first polymer precursor is partially or totally polymerized or crosslinked.

Clause 101. A compound of Formula I or Formula II: 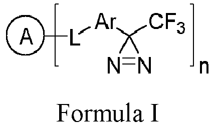 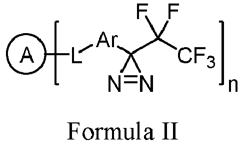 wherein in Formula I and Formula II:
A is a core moiety comprising one or more groups selected from optionally substituted alkyl, optionally substituted heteroalkyl, optionally substituted alkenyl, optionally substituted alkynyl, optionally substituted cycloalkyl, optionally substituted heterocycloalkyl, optionally substituted aryl, optionally substituted arylalkyl, optionally substituted heteroaryl, optionally substituted heteroarylalkyl, hydroxy, halo, cyano, trifluoromethyl, trifluoromethoxy, nitro, trimethylsilanyl, optionally substituted epoxide, optionally substituted glycidyl, optionally substituted acrylate, optionally substituted methacrylate, - OR^{a}, -SR^{a}, -OC(O)-R^{a}, -N(R^{a})₂, -C(O)R^{a}, -C(O)OR^{a}, -C(O)SR^{a}, -SC(O)R^{a}, -OC(O)OR^{a}, - OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a}, -S(O)ₜR^{a}, -S(O)ₜOR^{a}, -S(O)ₜN(R^{a})₂, -S(O)ₜN(R^{a})C(O)R^{a}, -O(O)P(OR^{a})₂, -O(S)P(OR^{a})₂, P(R^{a}-)₃, and Si, wherein R^{a} is independently selected at each occurrence from hydrogen, optionally substituted alkyl, optionally substituted heteroalkyl, optionally substituted alkenyl, optionally substituted alkynyl, optionally substituted cycloalkyl, optionally substituted heterocycloalkyl, optionally substituted aryl, optionally substituted arylalkyl, optionally substituted heteroaryl, and optionally substituted heteroarylalkyl;
L is at each independent occurrence a linker comprising one or more of -C₁-₁₀ alkyl-, - O-C₁-₁₀ alkyl-, -C₁-₁₀ alkenyl-, -O-C₁-₁₀ alkenyl-, -C₁-₁₀ cycloalkenyl-, -O-C₁-₁₀ cycloalkenyl-, -C₁-₁₀ alkynyl-, -O-C₁-₁₀ alkynyl-, -Ci-io aryl-, -O-C₁-₁₀-, -aryl-, -O-, -S-, -S(O)_{w}-, -C(O)-, -C(O)O-, -OC(O)-, -C(O)S-, -SC(O)-, -OC(O)O-, -N(R^{b})-, -N(R^{b})-C₁-₁₀ alkyl-, -C(O)N(R^{b})-, -N(R^{b})C(O)-, -OC(O)N(R^{b})-, -N(R^{b})C(O)O-, -SC(O)N(R^{b})-, -N(R^{b})C(O)S-, -N(R^{b})C(O)N(R^{b})-, -N(R^{b})C(NR^{b})N(R^{b})-, -N(R^{b})S(O)_{w}-, -S(O)_{w}N(R^{b})-, -S(O)_{w}O-, -OS(O)_{w}-, -OS(O)_{w}O-, -O(O)P(OR^{b})O-, (O)P(O-)₃, -O(S)P(OR^{b})O-, and (S)P(O-)₃, wherein w is 1 or 2, and R^{b} is independently hydrogen, optionally substituted alkyl, or optionally substituted aryl;
Ar is at each independent occurrence an optionally substituted aryl substituent, an optionally substituted arylalkyl, an optionally substituted heteroaryl, or an optionally substituted heteroarylalkyl; and
n is independently at each occurrence an integer from 0 to 7.

Clause 102. The compound of clause 101, wherein A comprises one or more phenyl groups.

Clause 103. The compound of clause 101, wherein A comprises one or more bi-phenyl groups.

Clause 104. The compound of clause 101, wherein A comprises a group selected from:

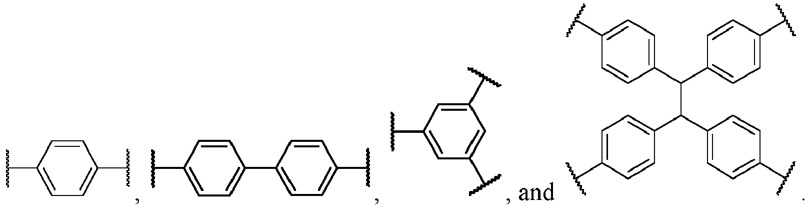

Clause 105. The compound of any one of clauses 101 to 104, wherein A comprises one or more C₁, C₂, C₃, or C₄ alkyl groups.

Clause 106. The compound of any one of clauses 101 to 104, wherein A comprises one or more Si.

Clause 107. The compound of any one of clauses 101 to 104, wherein A comprises one or more P.

Clause 108. The compound of any one of clauses 101 to 104, wherein A comprises one or more groups selected from

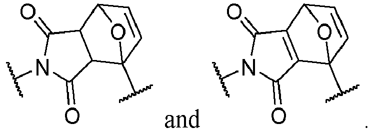

Clause 109. The compound of any one of clauses 101 to 107, wherein L comprises one or more groups selected from

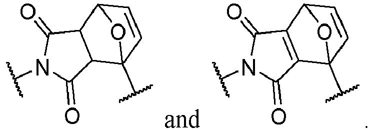

Clause 110. The compound of any one of clauses 101 to 108, wherein L comprises one or more C₁, C₂, C₃, or C₄ alkyl groups.

Clause 111. The compound of any one of clauses 101 to 108, wherein L comprises one or more groups selected from -CH₂-, -CH₂-CH₂-, and -CH₂-CH(CH₃)-.

Clause 112. The compound of any one of clauses 101 to 108, wherein L comprises one or more groups selected from -O-, -S-, -NH-, -C(O)NH-, -NHC(O)-, -NHC(O)NH-, and -S(O)₂NH-.

Clause 113. The compound of any one of clauses 101 to 112, wherein Ar is selected from

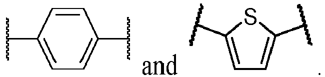

Clause 114. The compound of any one of clauses 101 to 112, the compound having formula 101, 102, 103, or 104:

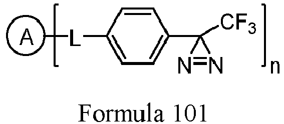

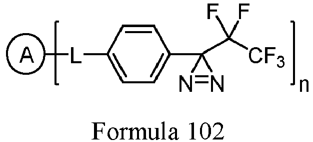

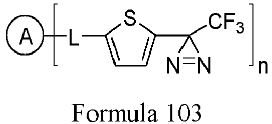

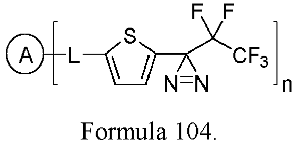

Clause 115. The compound of any one of clauses 101 to 112, the compound having Formula 105, 106, 107, 108, 109, 110, 111, 112, 113, 114, 115, or 116:

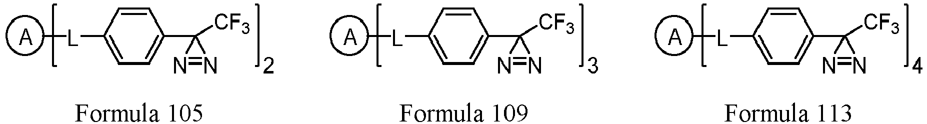

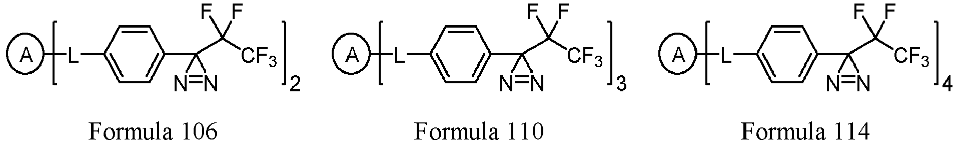

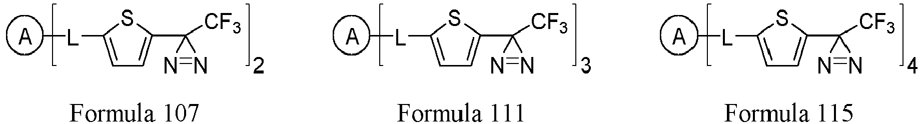

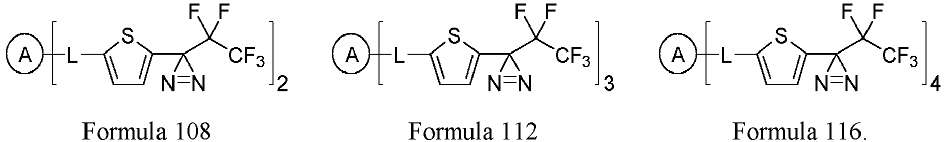

Clause 116. The compound of any one of clauses 101 to 115, the compound comprising at least one group selected from CH, CH₂, and CH₃.

Clause 117. The compound of any one of clauses 101 to 115, the compound comprising at least one CH group.

Clause 118. The compound of clause 101, wherein the compound has any one of Formulas 1001 to 1014:

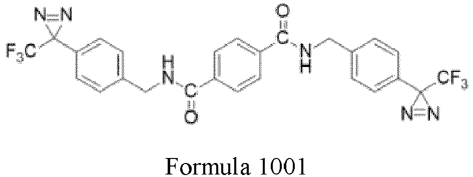

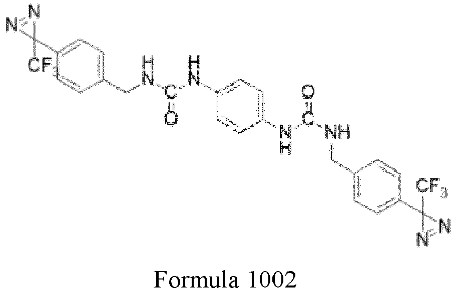

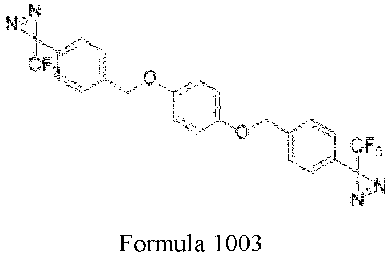

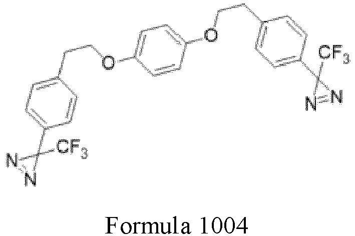

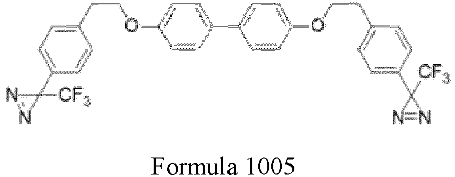

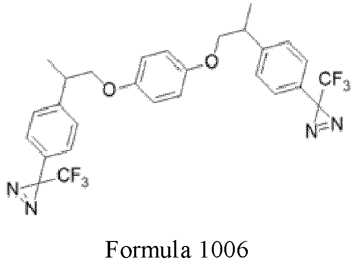

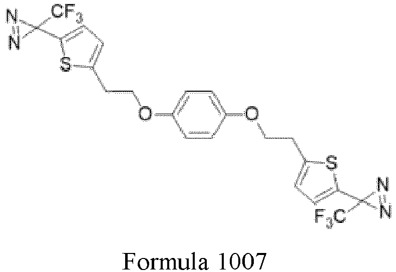

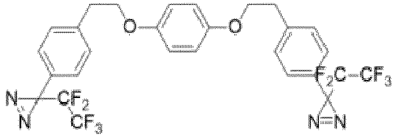

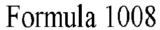

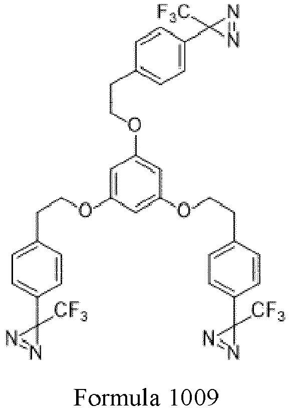

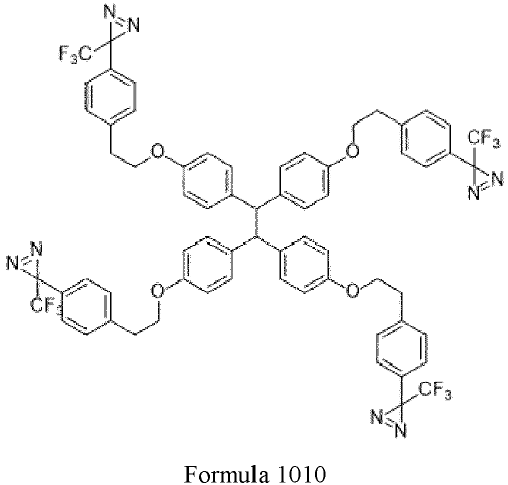

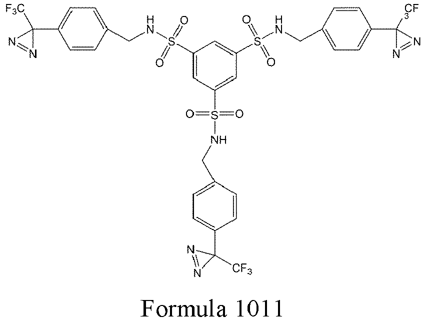

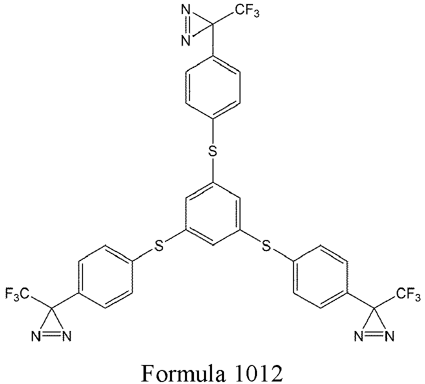

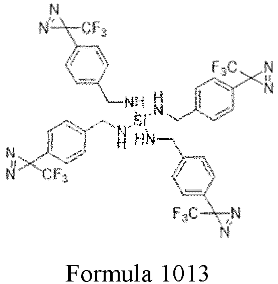

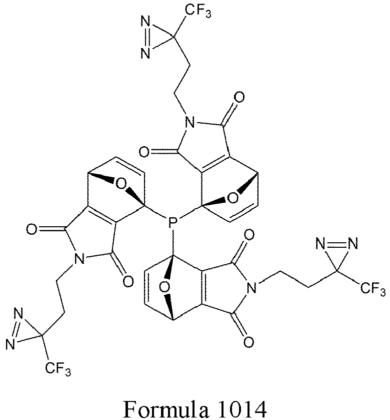

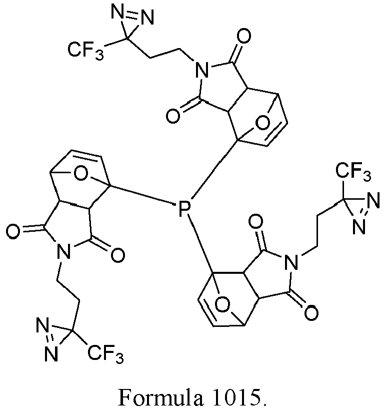

Clause 119. An adhesive comprising the compound of any one of clauses 101 to 118.

Clause 120. A resin mixture comprising a first polymer precursor comprising the compound of any one of clauses 101 to 118, and a second polymer precursor comprising a different compound comprising a polymerizable or crosslinkable group.

Clause 121. The resin mixture of clause 120, wherein the resin mixture further comprises a third polymer precursor comprising a different compound comprising a polymerizable or crosslinkable group.

Clause 122. The resin mixture of clause 120 or 121, wherein the second polymer precursor is partially or totally polymerized or crosslinked.

Clause 123. The resin mixture of any one of clauses 120 to 122, wherein the first polymer precursor is partially or totally polymerized or crosslinked.

Clause 124. The resin mixture of any one of clauses 121 to 123, wherein the third polymer precursor is partially or totally polymerized or crosslinked.

Clause 125. The resin mixture of any one of clauses 120 to 124, wherein the mixture comprises an elastomer.

Clause 126. A method of making the compound of any one of clauses 101 to 118, comprising reacting a first precursor comprising a diazirine group and a nucleophilic group with a second precursor comprising a leaving group.

Clause 127. A method of bonding two or more substrates, comprising contacting the substrates with the compound of any one of clauses 101 to 118.

Clause 128. A method of bonding two or more substrates, comprising contacting the substrates with the adhesive of clause 119.

Clause 129. A method of bonding two or more substrates, comprising contacting the substrates with the resin mixture of any one of clauses 120 to 124.

Clause 130. The method of any one of clauses 127 to 129, wherein at least one substrate comprises an elastomer.

Clause 131. The method of any one of clauses 127 to 130, further comprising exposure to an actinic radiation.

Clause 132. The method of clause 131, wherein the actinic radiation is patterned to bond selected interfacial areas between the substrates.

Clause 133. The method of any one of clauses 127 to 132, further comprising exposure to an elevated temperature.

Clause 134. The method of clause 133, wherein the elevated temperature is any temperature above room temperature and below the lowest decomposition temperature of any one of a substrate, the compound of any one of clauses 101 to 118, the adhesive of clause 119, or the resin mixture of any one of clauses 120 to 124.

Clause 135. The method of any one of clauses 127 to 134, further comprising washing a portion of unreacted compound of any one of clauses 101 to 118.

Clause 136. The method of any one of clauses 127 to 135, wherein a substrate comprises a silicone.

Clause 137. The method of any one of clauses 127 to 136, wherein a substrate comprises polydimethylsiloxane.

Clause 138. The method of any one of clauses 127 to 137, wherein a substrate is comprised in a fluidic elastomer actuator.

Clause 139. The method of any one of clauses 127 to 138, wherein a substrate comprises a polymer comprising at least one aliphatic moiety.

Clause 140. The method of clause 139, wherein the substrate comprises one or more of polyester, polyamide, polyaramid, polytetrafluoroethylene, polyethylene, polypropylene, polyurethane, silicone, polyethyleneglycol, polystyrene, polyethylene terephthalate, nylon, and LYCRA

Clause 141. The method of clause 139 or 140, wherein the substrate is comprised in a clothing garment.

Clause 142. The method of clause 141, wherein the clothing garment is a glove.

While preferred embodiments are shown and described herein, such embodiments are provided by way of example only and are not intended to otherwise limit the scope of the disclosure. Various alternatives to the described embodiments may be employed in practicing the disclosure.

A number of patent and non-patent publications are cited herein in order to describe the state of the art to which this disclosure pertains. The entire disclosure of each of these publications is incorporated by reference herein.

While certain embodiments are described and/or exemplified herein, various other embodiments will be apparent to those skilled in the art from the disclosure. The present disclosure is, therefore, not limited to the particular embodiments described and/or exemplified, but is capable of considerable variation and modification without departure from the scope and spirit of the appended claims.

### EXAMPLES

### Example 1: Tetraethyl-Orthosilicate Based Multifunctional Diazirine Adhesive

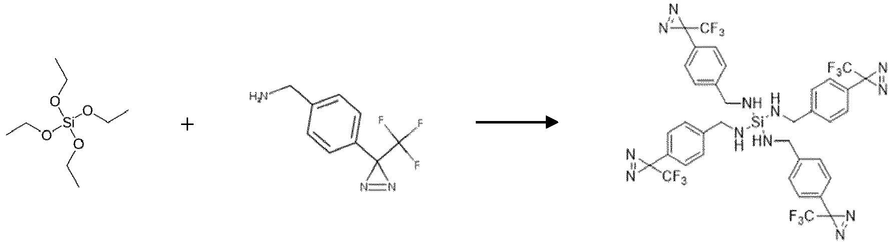

### Example 2: Multifunctional Diazirine Adhesive Based on Amine-Sulfonyl Chloride Reaction

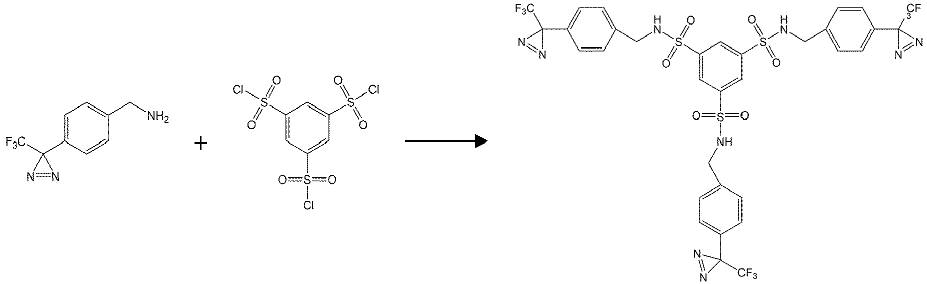

### Example 3: Multifunctional Diazirine Adhesive Based on Thiol-Bromo Click Chemistry

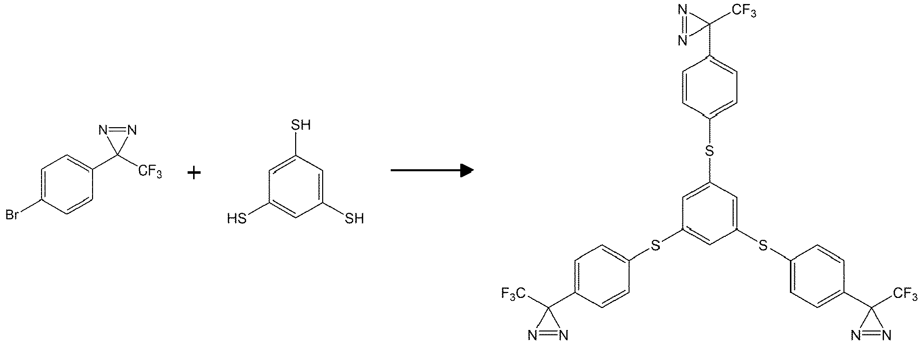

### Example 4: Multifunctional Diazirine Adhesive Based on Diels-Alder Reaction

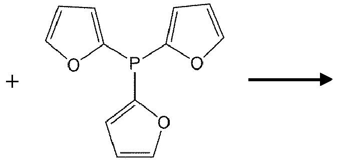

### Example 5: Bisdiazirine Derivatives With Varying Substitutions

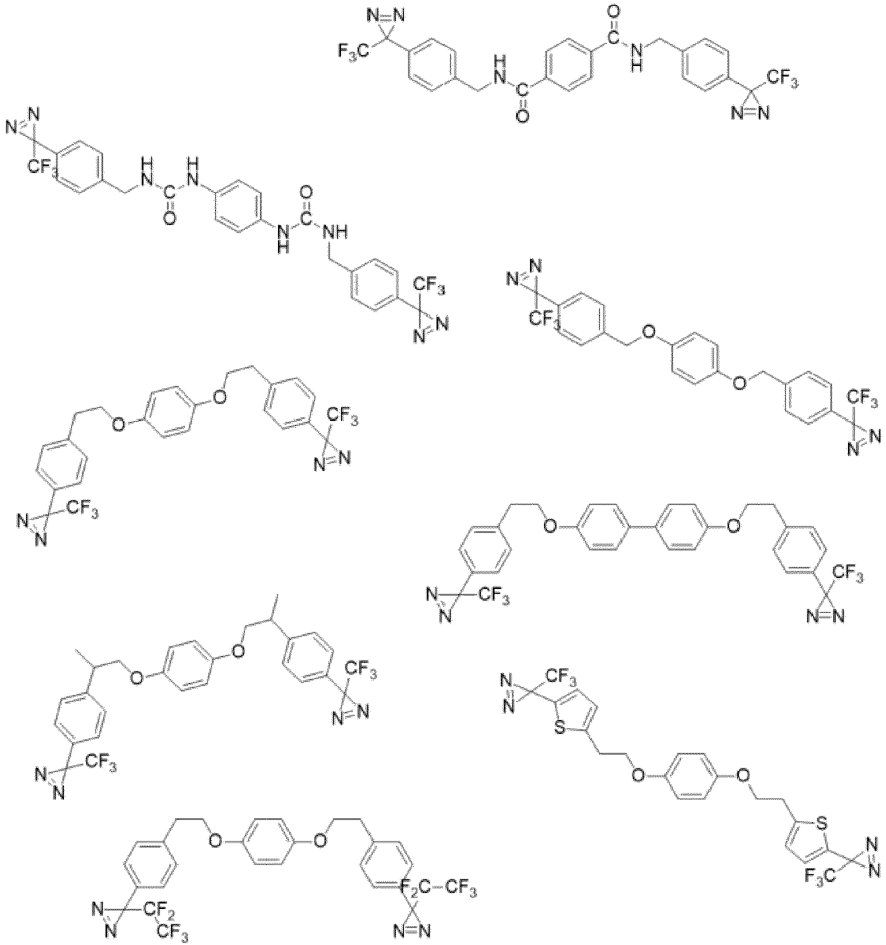

### Example 6: Multifunctional Diazirine With Varying Substitutions

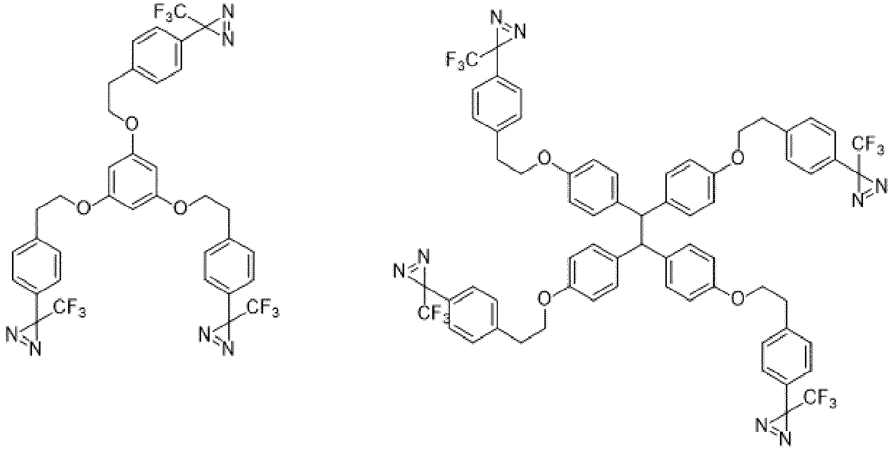

## Claims

1. A compound of Formula I or Formula II: 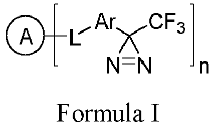 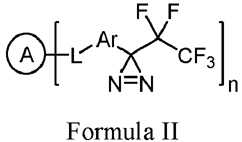 wherein in Formula I and Formula II:
A is a core moiety comprising one or more groups selected from optionally substituted alkyl, optionally substituted heteroalkyl, optionally substituted alkenyl, optionally substituted alkynyl, optionally substituted cycloalkyl, optionally substituted heterocycloalkyl, optionally substituted aryl, optionally substituted arylalkyl, optionally substituted heteroaryl, optionally substituted heteroarylalkyl, hydroxy, halo, cyano, trifluoromethyl, trifluoromethoxy, nitro, trimethylsilanyl, optionally substituted epoxide, optionally substituted glycidyl, optionally substituted acrylate, optionally substituted methacrylate, - OR^{a}, -SR^{a}, -OC(O)-R^{a}, -N(R^{a})₂, -C(O)R^{a}, -C(O)OR^{a}, -C(O)SR^{a}, -SC(O)R^{a}, -OC(O)OR^{a}, - OC(O)N(R^{a})₂, -C(O)N(R^{a})₂, -N(R^{a})C(O)OR^{a}, -N(R^{a})C(O)R^{a}, -N(R^{a})C(O)N(R^{a})₂, - N(R^{a})C(NR^{a})N(R^{a})₂, -N(R^{a})S(O)ₜR^{a}, -S(O)ₜR^{a}, -S(O)ₜOR^{a}, -S(O)ₜN(R^{a})₂, -S(O)ₜN(R^{a})C(O)R^{a}, -O(O)P(OR^{a})₂, -O(S)P(OR^{a})₂, P(R^{a}-)₃, and Si, wherein R^{a} is independently selected at each occurrence from hydrogen, optionally substituted alkyl, optionally substituted heteroalkyl, optionally substituted alkenyl, optionally substituted alkynyl, optionally substituted cycloalkyl, optionally substituted heterocycloalkyl, optionally substituted aryl, optionally substituted arylalkyl, optionally substituted heteroaryl, and optionally substituted heteroarylalkyl;
L is at each independent occurrence a linker comprising one or more of -C₁-₁₀ alkyl-, - O-C₁-₁₀ alkyl-, -C₁-₁₀ alkenyl-, -O-C₁-₁₀ alkenyl-, -C₁-₁₀ cycloalkenyl-, -O-C₁-₁₀ cycloalkenyl-, -C₁-₁₀ alkynyl-, -O-C₁-₁₀ alkynyl-, -C₁-₁₀ aryl-, -O-C₁-₁₀-, -aryl-, -O-, -S-, -S(O)_{w}-, -C(O)-, -C(O)O-, -OC(O)-, -C(O)S-, -SC(O)-, -OC(O)O-, -N(R^{b})-, -N(R^{b})-C₁-₁₀ alkyl-, -C(O)N(R^{b})-, -N(R^{b})C(O)-, -OC(O)N(R^{b})-, -N(R^{b})C(O)O-, -SC(O)N(R^{b})-, -N(R^{b})C(O)S-, -N(R^{b})C(O)N(R^{b})-, -N(R^{b})C(NR^{b})N(R^{b})-, -N(R^{b})S(O)_{w}-, -S(O)_{w}N(R^{b})-, -S(O)_{w}O-, -OS(O)_{w}-, -OS(O)_{w}O-, -O(O)P(OR^{b})O-, (O)P(O-)₃, -O(S)P(OR^{b})O-, and (S)P(O-)₃, wherein w is 1 or 2, and R^{b} is independently hydrogen, optionally substituted alkyl, or optionally substituted aryl;
Ar is at each independent occurrence an optionally substituted aryl substituent, an optionally substituted arylalkyl, an optionally substituted heteroaryl, or an optionally substituted heteroarylalkyl; and
n is independently at each occurrence an integer from 0 to 7.

2. The compound of claim 1, wherein A comprises one or more phenyl groups.

3. The compound of claim 1, wherein A comprises one or more bi-phenyl groups.

4. The compound of claim 1, wherein A comprises a group selected from:

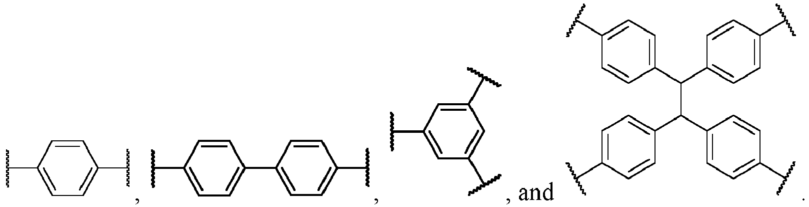

5. The compound of claim 1, wherein A comprises one or more C₁, C₂, C₃, or C₄ alkyl groups.

6. The compound of claim 1, wherein A comprises one or more Si.

7. The compound of claim 1, wherein A comprises one or more P.

8. The compound of claim 1, wherein A comprises one or more groups selected from

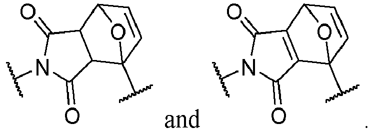

9. The compound of claim 1, wherein L comprises one or more groups selected from

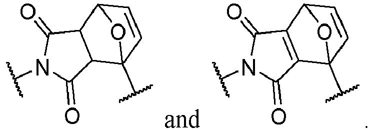

10. The compound of claim 1, wherein L comprises one or more C₁, C₂, C₃, or C₄ alkyl groups.

11. The compound of claim 1, wherein L comprises one or more groups selected from -CH₂-, -CH₂-CH₂-, and -CH₂-CH(CH₃)-.

12. The compound of claim 1, wherein L comprises one or more groups selected from -O-, -S-, -NH-, -C(O)NH-, -NHC(O)-, -NHC(O)NH-, and -S(O)₂NH-.

13. The compound of claim 1, wherein Ar is selected from 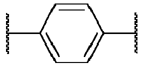 and

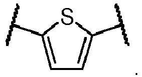

14. The compound of claim 1, the compound having formula 101, 102, 103, or 104:

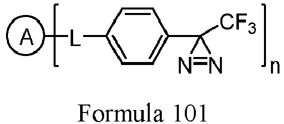

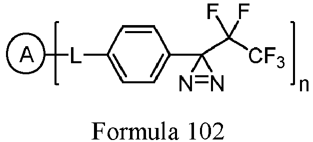

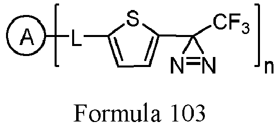

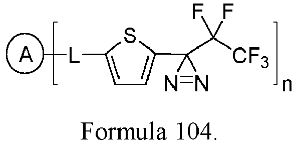

15. The compound of claim 1, the compound having Formula 105, 106, 107, 108, 109, 110, 111, 112, 113, 114, 115, or 116:

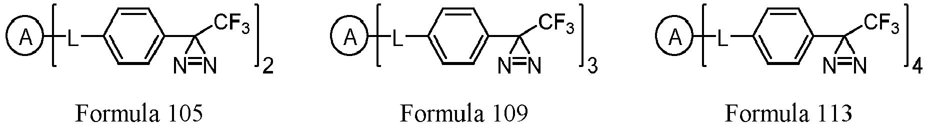

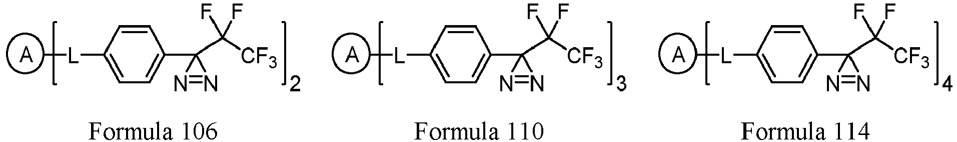

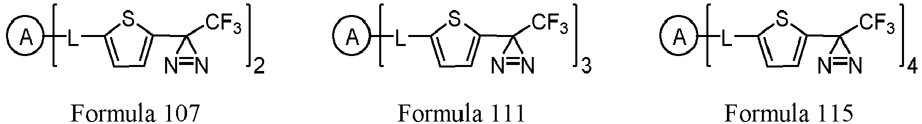

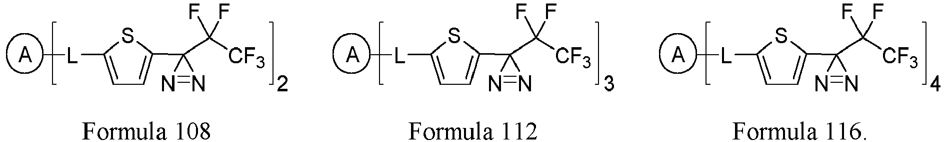

16. The compound of claim 1, the compound comprising at least one group selected from CH, CH₂, and CH₃.

17. The compound of claim 1, the compound comprising at least one CH group.

18. The compound of claim 1, wherein the compound has any one of Formulas 1001 to 1014:

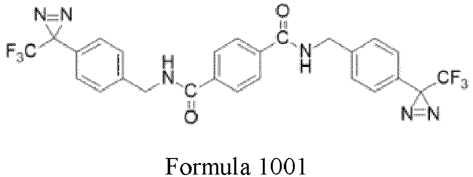

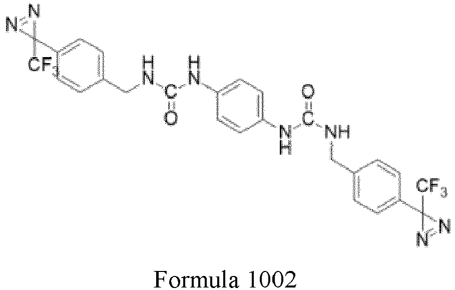

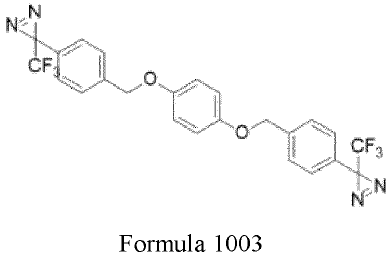

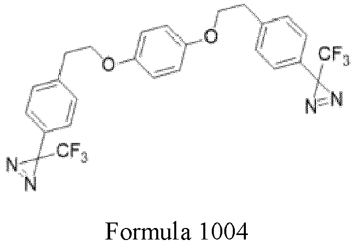

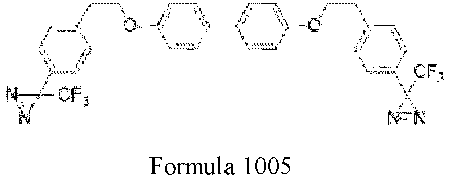

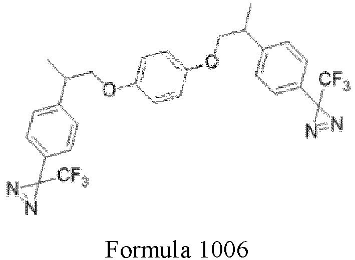

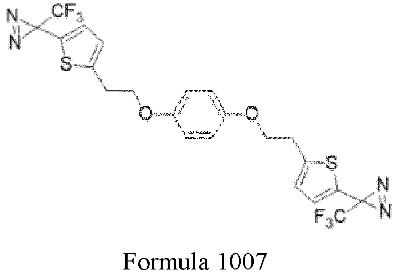

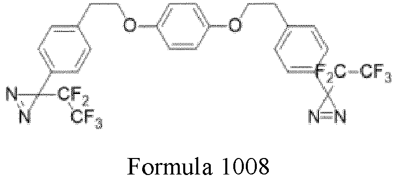

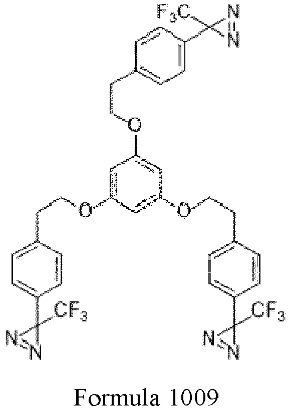

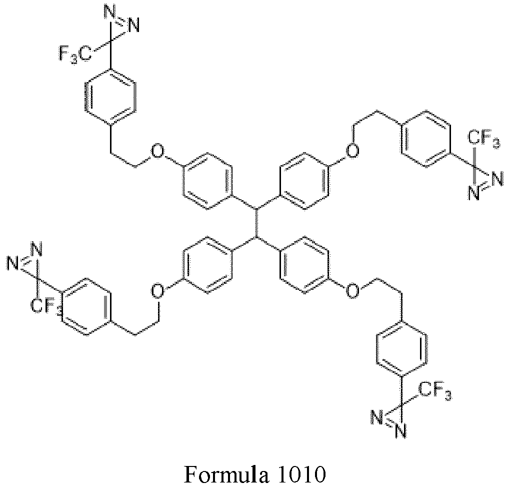

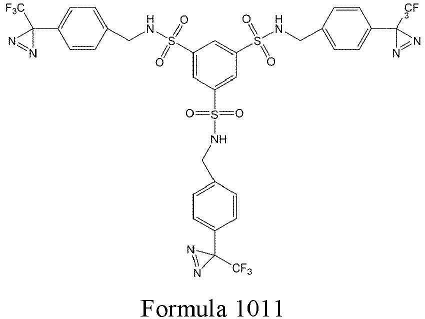

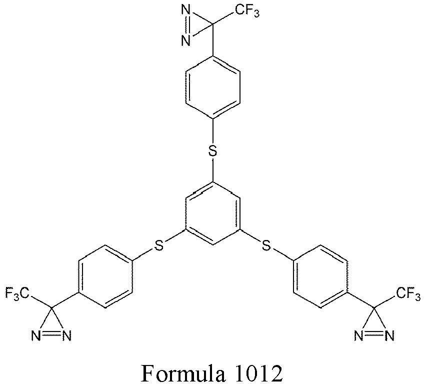

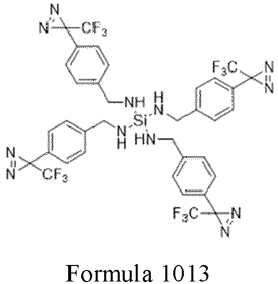

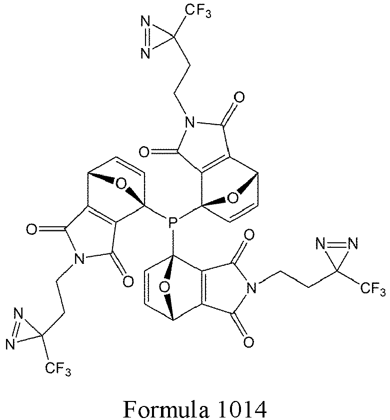

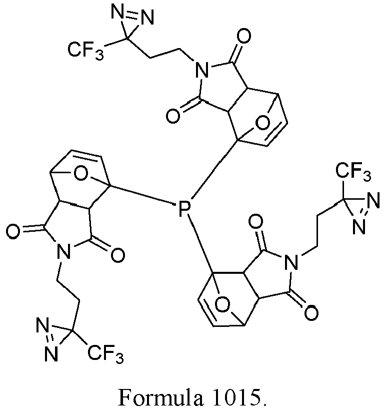

19. An adhesive comprising the compound of claim 1.

20. A method of bonding two or more substrates, comprising contacting the substrates with the compound of claim 1.
